# EUROPEAN PATENT APPLICATION

(11) **EP 2 033 968 A1**
(43) Date of publication of application: **11.03.2009**
(21) Application number: 07103379.9
(22) Date of filing: 02.03.2007
(51) Int. Cl.: C07K 14/435, A61K 31/436

(54) **Target of rapamycin modulators of and use thereof**

(71) Applicant: EPFL Ecole Polytechnique Fédérale de Lausanne, 1015 Lausanne (CH)
(72) Inventor: Brouard, Michel, CH-1024, Ecublens (CH); Barrandon, Yann, CH-1005, Lausanne (CH); AZZABI ZOURAQ, Fahd, CH-1005, Lausanne (CH)
(74) Representative: Leman Consulting S.A.

(57) **Abstract**

The present invention is related to new molecules capable of interacting with target of rapamycin (TOR) transcriptional complex. In particular, the present invention provides novel nucleic acid molecules, processes for production thereof, kits containing thereof, use of these in an assay for the identification of mammalian target of rapamycin (mTOR) modulators, use of mammalian target of rapamycin (mTOR) modulators identified by this assay for the preparation of a formulation for the control of hair growth and compositions for the control of hair growth.

## Description

### Field of the Invention

The present invention relates to new nucleic acid molecules capable of interacting with target of rapamycin (TOR) transcriptional complex. In particular, the invention provides new nucleic acid molecules useful in an assay for the identification of mammalian target of rapamycin (mTOR) modulators and a use of mammalian target of rapamycin (mTOR) modulators for the preparation of a formulation in the control of hair growth. More particularly, the present invention provides novel nucleic acid molecules, processes for production thereof, kits containing thereof, use of these in an assay for the identification of mammalian target of rapamycin (mTOR) modulators, use of mammalian target of rapamycin (mTOR) modulators identified by this assay for the preparation of a formulation for the control of hair growth and compositions for the control of hair growth.

### Background of the Invention

Target of rapamycin (TOR), first identified in *Saccharomyces cerevisiae,* is a serine/threonine kinase that regulates cell growth in response to rapamycin or environmental cues, e.g. nutrients, hypoxia or growth factors *(*Martin and Hall, 2005, Curr Opin Cell Biol 17, 158-166). TOR elicits a cellular response by regulating translation and has been recently demonstrated to interact with the transcription of ribosomal genes through the forkhead-like transcription factor Fhl1 (Martin et al., 2004, Cell 119, 969-979*).* Importantly, TOR cytoplasmic signaling pathway is conserved from yeast to mammals, albeit with significant differences; for instance, there are two different TOR (TOR1 and TOR2) in yeast, whereas mammals have one (mTOR). mTOR associates with a number of cytoplasmic proteins to form a rapamycin-sensitive complex (mTORC1) and a rapamycin-insensitive complex (mTORC2). mTORC1 contains mTOR, the rapamycin-binding protein FKBP12, raptor and mLST8, whereas mTORC2 contains mTOR, rictor and mLST8 *(Martin and Hall, 2005, above)*. Mammalian cells respond to growth factors, e.g. insulin or insulin-like growth factors, by activating the phosphatidylinositol 3-kinase (PI3K) and Akt pathways that are wired to mTORC1 signaling through the tuberous sclerosis proteins TSC1 (hamartin) and TSC2 (tuberin). Cells also respond to nutrients, energy and environmental stress (hypoxia) through mTORC1 that acts by increasing translation via phosphorylation of S6K1 and 4E-BP, by ribosome biogenesis and possibly regulation of transcription. mTORC2 is insensitive to rapamycin and controls spatial aspects of cell growth, possibly through regulation of the actin cytoskeleton (Jacinto et al., 2004, Nat Cell Biol 6, 1122-1128); mTORC2 can also phosphorylate Akt at Ser473 (Jacinto et al., 2006, Cell 127, 125-137*).* Importantly, mTOR has been implicated in many diseases including cancer and hamartoma syndromes (tuberous sclerosis), metabolic diseases including type 2 diabetes and obesity, cardiovascular diseases, allograft rejection and autoimmune disorders.

Rapamycin is a bacterial macrolide with antifungal and immunosuppressant activities *(*Dumont et al., 1990, J Immunol. 144:251) that is known to modulate TOR signaling pathway. Inactivation of the TOR proteins or rapamycin treatment mimics nutrient deprivation in yeast, Drosophila and mammalian cells.

The epidermis, the skin outermost layer, is a squamous epithelium that is constantly exposed to a varying environment to which it must adapt. Most importantly, it is continuously renewed and contains keratinocyte stem cells, also known as holoclones, that are distributed throughout the basal layer of the epithelium *(*Blanpain and Fuchs, 2006, Annu Rev Cell Dev Biol 22, 339-373*).* Each keratinocyte stem cell is in charge of a limited portion of epidermis, which appears as an assembly of functionally independent columnar units. It is thought that epidermal stem cells divide infrequently and asymmetrically to generate daughter stem cells and transient amplifying cells with limited potential. The latter cells then actively divide to compensate for the differentiated cells that are continuously sloughed off from the epithelial surface. Keratinocyte stem cells, because of their unique location at the interface of the body with the outside world, need to constantly adapt to a variable microenvironment, which includes adjusting to varying temperature and pH, nutrients and oxygen availability, shear stress and wounding. For instance, stem cells that are located in a 100 to 150 µm thick epidermis are, of all tissue stem cells, the most exposed to external temperature fluctuation.

Keratinocyte stem cells are also present in hair follicles, to which renewal they contribute *(*Claudinot et al., 2005, Proc Natl Acad Sci U S A 102, 14677-14682*).* Keratinocyte stem cells, whether from the epidermis or the hair follicles, can efficiently migrate can be efficiently expanded in culture and undergo more than 180 doublings under appropriate conditions *(*Mathor et al., 1996, Proc Natl Acad Sci US A 93, 10371-10376*).* Importantly, they retain the capacity to permanently engraft if transplanted in a proper environment, a property that has major clinical implications and can be life saving.

As cells and in particular stem cells such as keratinocyte stem cells respond to a variety of physical or chemical signals (e.g. temperature, microwaves, pH, hypoxia, nutrients, growth factors, thermal stress etc...) by exquisite tuning of translation and transcription of a number of developmentally regulated genes through mTOR, it would be highly desirable to identify molecules inferring with DNA binding of mTOR transcriptional complexes to define new therapeutic approaches by fine-tuning proliferation, migration and differentiation of stem cells and diseased stem cells.

### Summary of the Invention

The present invention is directed towards new nucleic acid molecules capable of interacting with target of rapamycin (TOR) transcriptional complex. In particular, the invention provides new nucleic acid molecules, processes for production thereof, kits containing thereof, use of these in an assay for the identification of mammalian target of rapamycin (mTOR) modulators, use of mammalian target of rapamycin (mTOR) modulators identified by this assay for the preparation of a formulation for the control of hair growth and compositions for the control of hair growth.

A first aspect of the invention provides a nucleic acid molecule characterized by a sequence having at least 80% identity or homology (such as at least 85%, at least 90%, at least 95%, at least 98%) with a sequence of nucleic acid selected from the group consisting of: SEQ ID NO: 1; SEQ ID NO: 2; SEQ ID NO: 3; SEQ ID NO: 4; SEQ ID NO: 5; SEQ ID NO: 6 and SEQ ID NO: 7.

A second aspect of the invention relates to a nucleic acid molecule characterized by a sequence which is a complement of a nucleic acid having at least 80% identity or homology (such as at least 85%, at least 90%, at least 95%, at least 98%) selected from the group consisting of : SEQ ID NO: 1; SEQ ID NO: 2; SEQ ID NO: 3; SEQ ID NO: 4; SEQ ID NO: 5; SEQ ID NO: 6 and SEQ ID NO: 7.
A third aspect of the invention resides in a nucleic acid molecule having a sequence comprising at least two nucleic acid molecule according to the invention.
A fourth aspect of the invention relates to a purified and isolated DNA molecule comprising a polynucleotide sequence having at least 80% identity or homology (such as at least 85%, at least 90%, at least 95%, at least 98%) to a nucleic acid sequence selected from the group consisting of: SEQ ID NO: 1; SEQ ID NO: 2; SEQ ID NO: 3; SEQ ID NO: 4; SEQ ID NO: 5; SEQ ID NO: 6 and SEQ ID NO: 7; and wherein the nucleic acid sequence is transcriptionally linked to a promoter.
A sixth aspect of the invention is an isolated nucleic acid molecule comprising the nucleic acid molecule according to the invention and a nucleic acid sequence conferring the activity of a reporter gene.
A seventh aspect of the invention relates to an expression vector comprising the nucleic acid molecule according to the invention.
An eighth aspect of the invention is a host cell transfected or transformed with an expression vector according the invention or a nucleic acid according to the invention.
A ninth aspect of the invention is a process for the preparation of a transgenic host comprising the steps of transfecting a host cell with the nucleic acid molecule according to the invention or with a vector according to the invention.
A tenth aspect of the invention provides a kit comprising at least one nucleic acid molecule according to the invention.
An eleventh aspect of the invention relates to a process for the identification of modulators of the mTOR signaling pathway comprising the steps of:
(a) Providing a nucleic acid molecule according to the invention;
(b) Providing a cell or plurality of cells in a sample;
(c) Transfecting cell or plurality of cells with the nucleic acid provided in (a);
(d) Incubating said cell, or plurality of cells, with at least one putative modulator agent;
(e) Measuring the transcription level of the nucleic acid provided in (a);
(f) Comparing the transcription level measured in (e) or with the transcription level of the nucleic acid provided in (a) in the absence of putative modulator agent;
(g) Selecting the modulating compound(s).
A twelfth aspect of the invention is a use of a mTOR signaling pathway modulator for the preparation of a composition for the control of hair growth.
A thirteenth aspect of the invention is a use of a mTOR signaling pathway modulator for the preparation of a composition for the control of stem cells growth.
A fourteenth aspect is a process for the preparation of epithelium grafts comprising the steps of:
(a) Providing a cell or plurality of cells in a sample;
(b) Incubating said cell, or plurality of cells, with at least one mTOR modulator;
(c) Harvesting the cells after incubation.
A fifteenth aspect is a cosmetic composition comprising Rapamycin or an analogue thereof and a cosmetically acceptable carrier or vehicle for topical application.
Other features and advantages of the invention will be apparent from the following detailed description.

### Description of the figures

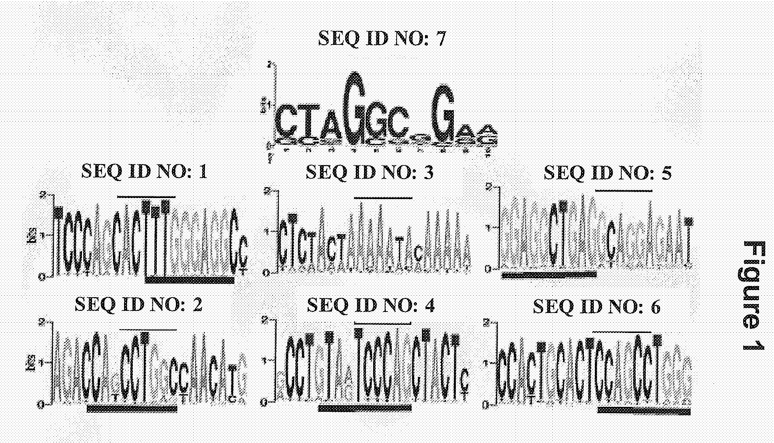

Figure 1 shows nucleic acid sequences of the invention of SEQ ID NO: 1 to SEQ ID N: 7 and some homologs thereof represented as "logo sequences" *(*Crooks et al., 2004, Genome Research, 14:1188-1190*).* In this representation, each logo consists of stacks of symbols, one stack for each position in the sequence. The overall height of the stack (expressed in bits) indicates the sequence conservation at that position, while the height of symbols (expressed in bits) within the stack indicates the relative frequency of each amino or nucleic acid at that position, 2 bits corresponding to a frequency of 100%.
Figure 2 shows that a minimal sequence (SEQ ID NO: 19 in the upstream sequence of CXCL14 of SEQ ID NO: 22) is enough to confer temperature sensitivity human CXCL14 gene (NT_034772.5, SEQ ID NO: 32).
Figure 2A shows the different plasmid constructions (1) to (4) from Example 2 and the portions (i) of the original sequence (SEQ ID NO: 19) that have been mutated as indicated in (ii) to lead to the mutated sequence of SEQ ID NO: 20;
Figure 2B shows the luciferase relative activity (LRA) observed with the plasmid constructions (1) to (4) at 36°C (white) and 37°C (black).
Figure 2C shows the mTOR enrichment (F) of the nucleus by chromatin immunoprecipitation analysis such as described in Example 3 when cells are transfected with plasmid construction (1), (2) or (3). Plasmid constructions are as follows: (1): HPV16 promoter alone (control), (2): SEQ ID NO: 19-HPV16 promoter, (3): SEQ ID NO: 20 (i.e. mutated SEQ ID NO: 19)-HPV16 promotor and SEQ ID NO: 20 alone (control) (4).
Figure 3 shows the plating efficiency of rapamycin-treated keratinocyte stem cells (+) as described in Example 3 as compared to non-treated keratinocyte stem cells (-).

### Detailed Description of the invention

The term "mTOR modulator" as used herein refers to molecules or compounds that modulate the mTOR signaling function such as the ability if activating or repressing gene expression allowing cells to adjust to a change of environment, in particular stem cells. In the context of the invention, the term mTOR modulator, encompasses modulators of mTOR proteins (e.g. modulators of the mTOR signaling pathway). The term mTOR modulator can be an inhibitor or an antagonist or an agonist. Examples of mTOR modulator includes rapamycin, also called sirolimus, everolimus, temsirolimus and analogs of sirolimus (i.e. rapamycin and derivatives thereof). For example, sirolimus has many analogs that are substituted at either the 2-, 7-or 32-positions. Examples of sirolimus analogues are cited in Ritacco et al., 2005, Appl. Environ. Microbiol, 71, 1971-1976*.* The skilled person would understand that the term "derivative" is used herein interchangeably with term "analog".
The term "inhibitor" is defined as a molecule that inhibits or alter completely or partially the activity of a biological molecule.
The term "agonist" is defined is defined as a molecule that agonizes or increases the activity of biological molecule.

The term "antagonists" is defined as a molecule that antagonizes completely or partially the activity of biological molecule.
The term "mTOR antagonist" comprises all antagonists of all suitable forms mTOR described herein that antagonize one or more biological activity of mTOR or mTOR proteins. Examples of mTOR inhibitor or antagonist in the context of the invention are rapamycin, the tuberous sclerosis proteins TSC1 (hamartin) and TSC2 (tuberin) such as described in Gao et al., 2002, Nat Cell Biol, 4, 699-704*.*
For example, the mTOR antagonists of the invention are able to antagonize the ability of mTOR to participate to the interactions with cytoplasmic proteins within a transcriptional complex. The term "mTOR antagonist" includes but is not limited to: mTOR specific antibodies of any sort (polyclonal, monoclonal, antibody fragments, antibody variants), chimaeric proteins, natural or unnatural proteins with mTOR antagonizing activities, small molecules, nucleic acid derived polymers (such as DNA and RNA aptamers, PNAs or LNAs), peptidomimetics, fusion proteins, or gene therapy vectors driving the expression of such mTOR antagonists.
The term "mTOR protein" refers to mTOR and the cytoplasmic proteins within the mTOR transcriptional complex such as RAPTOR, RICTOR, mLST8 such as described in *Martin et Hall, 2005,* above.
The term "complement" used in the context of a nucleic acid sequence of the invention is defined by the replacement of each base by its complementary base: adenine (A) by thymidine (T), cytosine (C) by guanine (G), and vice versa.
The term "rapamycin" as used herein refers to a compound represented by the drug sirolimus. Rapamycin is an antifungal antibiotic which may be naturally extracted from a streptomycetes, e.g. Streptomyceshygroscopicus, chemically synthesized or produced by genetic engineering cell culture techniques.
The term "analog" as used herein, refers to any compound having substantial structure-activity relationships to a parent compound such that the analog has similar biochemical activity as the parent compound.
The term "control of hair growth" comprises the promotion of hair growth or the inhibition of hair growth.

The term "promotion of hair growth" includes the reduction or the slowing of the rate of hair loss and/or the treatment of hair loss conditions.
The term "hair loss" conditions include male pattern alopecia (androgenic alopecia), female pattern hair loss, common pattern baldness, alopecia senilis, alopecia areata, telogen effluvium, diseases accompanied by basic skin lesions or tumors, and systematic disorders such as nutritional disorders and internal secretion disorders. Hair loss can be attributed to aging, genetic disposition, the activation of male hormones, the loss of blood supply to hair follicles, and scalp abnormalities.
The term "alopecia" results when the pilar cycle is disturbed and refers to deficient hair growth and partial or complete loss of hair, including without limitation androgenic alopecia (male pattern baldness), toxic alopecia, alopecia senilis, alopecia areata, and trichotillomania. Alopecia areata usually presents as varying amounts of patchy hair loss, most commonly on the scalp (though it can affect any hair-bearing surface), but may also manifest as larger patches with little or no hair. Related forms of the disease include: alopecia totalis, characterized by complete loss of all scalp hair and alopecia universalis, characterized by loss of all body hair.
The most frequent phenomenon is a shortening of the hair growth or anagen phase due to cessation of cell proliferation. This results in an early onset of the catagen phase, and consequently a large number of hairs in the telogen phase during which the follicles are detached from the dermal papillae and the hairs fall out. Alopecia has a number of etiologies, including genetic factors, aging, local and systemic diseases, febrile conditions, mental stresses, hormonal problems, and secondary effects of drugs.
Androgenic alopecia affects both men and women, although women tend to lose less hair, and in a more diffuse pattern than men. There is also evidence that androgenic hormones, such as testosterone, coupled with a genetic predisposition, are necessary for the development of male pattern baldness. Female pattern baldness is thought to result from a decrease in estrogen, a hormone that normally counteracts the balding effect of testosterone, although there is so far no consensus on whether pattern baldness in women is truly androgen-dependent.

Telogen effluvium manifests as excessive shedding of hair, which occurs as cycling hair follicles prematurely enter the resting phase of the hair growth cycle, called telogen. It may be precipitated by a multitude of stress-related causes, including high fevers, childbirth, severe infections, severe chronic illness, severe psychological stress, major surgery, an over- or under-active thyroid gland, crash diets with inadequate protein, and a variety of medications, including, e.g. retinoids, beta blockers, calcium channel blockers, antidepressants and non-steroidal anti-inflammatories, including ibuprofen and acetominophen. In contrast, anagen effluvium, the most common type of chemotherapy-induced alopecia, results from the abrupt cessation of mitotic activity in hair matrix cells of anagen hair follicles. This induces the follicles to produce either no hair, or produce only narrow defective hair sheaths which are predisposed to fracture and loss. This type of alopecia can be seen to some degree in most anti-neoplastic therapies, depending on dosage and route of administration.
The treatment options for hair loss condition are limited and include hair transplants, hormonal supplementation, topical solution of minoxidil, a drug that affects calcium homeostasis, corticosteroids (e.g. prednisone, dexamethasone or hydrocortisone) administered orally, topically, or by injection, with oral finasteride.
The term "inhibition of hair growth" includes the reduction or the slowing of the rate of hair growth and/or the reduction and/or removal of unwanted hairs on the body, essentially for cosmetic reasons. Conventional procedures generally used to remove unwanted hair, including shaving, electrolysis, depilatory creams or lotions, waxing, plucking, and therapeutic antiandrogens but have drawbacks associated with them. Shaving, for instance, can cause nicks and cuts, and can leave a perception of an increase in the rate of hair regrowth. Shaving also can leave an undesirable stubble. Electrolysis, on the other hand, can keep a treated area free of hair for prolonged periods of time, but can be expensive, painful, and sometimes leaves scarring. Depilatory creams, though very effective, typically are not recommended for frequent use due to their high irritancy potential. Waxing and plucking can cause pain, discomfort, and poor removal of short hair. Finally, antiandrogens - which have been used to treat female hirsutism - can have unwanted side effects.

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e. arresting or slowing its development; or relieving the disease, i.e. causing regression of the disease and/or its symptoms or conditions.
The term "subject" as used herein refers to mammals. For examples, mammals contemplated by the present invention include human, primates, domesticated animals such as cattle, sheep, pigs, horses and the like.
The term "topical" means a composition of mTOR modulator such as a mTOR inhibitor such as rapamicyn and analog thereof incorporated in a suitable pharmaceutical carrier which can be applied to the treatment site for local action.
The term "isolated" is used to indicate that the molecule is free of association with other proteins or polypeptides, for example as a purification product of recombinant host cell culture or as a purified extract.

### Nucleotide sequences

The isolated nucleotide sequences according to the invention include the nucleic acid presented in SEQ ID NO: 1; SEQ ID NO: 2; SEQ ID NO: 3; SEQ ID NO: 4; SEQ ID NO: 5; SEQ ID NO: 6 and SEQ ID NO: 7 and variants thereof
A "variant" as referred to herein, means an isolated nucleic acid sequence homologous or substantially homologous to an original sequence selected from the group consisting of SEQ ID NO: 1; SEQ ID NO: 2; SEQ ID NO: 3; SEQ ID NO: 4; SEQ ID NO: 5; SEQ ID NO: 6 and SEQ ID NO: 7, but which has a nucleic acid sequence different from that original sequence because of one or more substitutions. "Substantially homologous" means a variant nucleic acid sequence that is at least 80%, at least 85%, at least 90%, at least 95%, at least 96%, at least 97%, at least 98% or at least 99% identical to the original sequences, as disclosed above. The percent identity of two nucleic acid sequences can be determined by visual inspection and/or mathematical calculation, or more easily by comparing sequence information using a computer program such a BLAST software such as described in McGinnis et al., 2004, Nucleic Acid Res., 32, W20-W25*.* Variants according to the invention may comprise a sequence having at least one substituted nucleic acid.
The isolated nucleotide sequences according to the invention include the nucleic acid molecule characterized by a sequence which is a complement of a nucleic acid sequence presented in SEQ ID NO: 1; SEQ ID NO: 2; SEQ ID NO: 3; SEQ ID NO: 4; SEQ ID NO: 5, SEQ ID NO: 6 and SEQ ID NO: 7 and variants thereof
The isolated nucleotide sequences according to the invention further includes isolated nucleic acid molecules having a sequence comprising at least two nucleic acid molecules each characterized by a sequence having at least 80% identity or homology (such as at least 85%, at least 90%, at least 95%, at least 98%) with a sequence of nucleic acid selected from the group consisting of: SEQ ID NO: 1; SEQ ID NO: 2; SEQ ID NO: 3; SEQ ID NO: 4; SEQ ID NO: 5 and SEQ ID NO: 6 and their complement.
The invention further includes purified and isolated DNA molecules comprising a polynucleotide sequence having at least 80% identity or homology (such as at least 85%, at least 90%, at least 95%, at least 98%) to a nucleic acid sequence according to the invention and wherein the nucleic acid sequence is transcriptionally linked to a promoter.
The isolated nucleotide sequences according to the invention include isolated nucleic acid molecules comprising the nucleic acid molecule according to the invention and a nucleic acid sequence conferring the activity of a reporter gene such as luciferase or the like.

### Brief description of the sequence listing

Table 1 below presents the sequence identity numbers and associated molecules:

**Table 1**

| SEQ ID NO. | Molecule |
|---|---|
| 1 | DNA sequence for motif 1 |
| 2 | DNA sequence for motif 2 |
| 3 | DNA sequence for motif 3 |
| 4 | DNA sequence for motif 4 |
| 5 | DNA sequence for motif 5 |
| 6 | DNA sequence for motif 6 |
| 7 | DNA sequence for motif 7 |
| 8 | DNA "consensus" sequence for motif 1 |
| 9 | DNA "consensus" sequence for motif 2 |
| 10 | DNA "consensus" sequence for motif 3 |
| 11 | DNA "consensus" sequence for motif 4 |
| 12 | DNA "consensus" sequence for motif 5 |
| 13 | DNA "consensus" sequence for motif 6 |
| 14 | DNA "consensus" sequence for motif 7 |
| 15 | DNA sequence n° 1 containing at least 2 motifs selected from 1 to 6 |
| 16 | DNA sequence n° 2 containing at least 2 motifs selected from 1 to 6 |
| 17 | DNA sequence n° 3 containing at least 2 motifs selected from 1 to 6 |
| 18 | DNA sequence n° 4 containing at least 2 motifs selected from 1 to 6 |
| 19 | DNA sequence n°5 containing at least 2 motifs selected from 1 to 6 |
| 20 | DNA sequence containing mutated SEQ ID NO: 19 |
| 21 | DNA sequence n° 6 containing at least 2 motifs selected from 1 to 6 |
| 22 | cDNA of CXCL14 |
| 23 | RT PCR primer for CXCL14 |
| 24 | RT PCR primer for CXCL14 |
| 25 | cDNA of DNAJB5 |
| 26 | RT PCR primer for DNAJB5 |
| 27 | RT PCR primer for DNAJB5 |
| 28 | RT PCR primer for GAPDH |
| 29 | RT PCR primer for GAPDH |
| 30 | RT PCR primer for 18S |
| 31 | RT PCR primer for 18S |
| 32 | CXCL 14 human gene (NT_034772) |
| 33 | pGL3b plasmid |
| 34 | HPV16 promoter |
| 35 | SV40 promoter |
| 36 | pRL-SV40 plasmid |
| 37 | EGFR human gene (NT_033968) |

One process for producing according to the invention comprises culturing a host cell transformed with an expression vector comprising a DNA sequence that encodes a sequence according to the invention under conditions sufficient to promote expression of encodes a sequence according to the invention. The sequence according to the invention is then recovered from culture medium or cell extracts, depending upon the expression system employed. As known to the skilled artisan, procedures for purifying a recombinant protein will vary according to such factors as the type of host cells employed and whether or not the recombinant protein is secreted into the culture medium.
A desired DNA sequence may be chemically synthesized using techniques known per se. DNA fragments also may be produced by restriction endonuclease digestion of a full length cloned DNA sequence, and isolated by electrophoresis on agarose gels. Linkers containing restriction endonuclease cleavage site(s) may be employed to insert the desired DNA fragment into an expression vector, or the fragment may be digested at cleavage sites naturally present therein.

Typically, the efficacy of the mTOR modulators identified by the assay according to the invention may be assayed in various assays such as described in Example 4, hair follicle growth assay such as described in WO 2006/105359, but also dissociated cells or organotypic cultures of hair follicles.

### Assay

The process according to the invention is a process for the identification of modulators of the mTOR signaling pathway comprising the steps of:
(a) Providing a nucleic acid molecule according to the invention;
(b) Providing a cell or plurality of cells in a sample;
(c) Transfecting cell or plurality of cells with the nucleic acid provided in (a);
(d) Incubating said cell, or plurality of cells, with at least one putative modulator agent;
(e) Measuring the transcription level of the nucleic acid provided in (a);
(f) Comparing the transcription level measured in (e) or with the transcription level of the nucleic acid provided in (a) in the absence of putative modulator agent;
(g) Selecting the modulating compound(s).
Typically, the assay may be carried out wherein the isolated nucleic acid molecule provided in step (a) comprises the nucleic acid molecule according to the invention and a nucleic acid sequence conferring the activity of a reporter gene, such as for example luciferase such as described in Example 2.
Cells that may be used in the assay include stem cells such as keratinocyte stem cells, foetal stem cells, adult somatic stem cells, non-human embryonic stem cells, hematopoietic cells or gut stem cells and spermatogonia cells.

### Compositions

The invention provides pharmaceutical, cosmetic or therapeutic agents as compositions and methods for treating a patient, preferably a mammalian patient, and most preferably a human patient who is suffering from a medical disorder, and in particular a hair growth disorder. Pharmaceutical compositions of the invention can contain one or more mTOR modulator (including an inhibitor such as rapamycin or an analog thereof, agonist or antagonist) in any form described herein. Compositions of this invention may further comprise one or more pharmaceutically or cosmetically acceptable additional ingredient(s) such as alum, stabilizers, antimicrobial agents, buffers, coloring agents, flavoring agents, adjuvants, and the like. The vehicle can be inert or can possess cosmetic, physiological and/or pharmaceutical benefits of its own. mTOR modulators, together with a conventionally employed adjuvant, carrier, diluent or excipient may be placed into the form of pharmaceutical or cosmetic compositions, preferably for topical use and unit dosages thereof, and in such form may be employed as liquids such as solutions, suspensions, emulsions, elixirs, gels in the form of topical solutions for external use or in the form of sterile injectable solutions for subcutaneous injection, preferably in the form of topical solutions for external use. Such pharmaceutical or cosmetic compositions and unit dosage forms thereof may comprise ingredients in conventional proportions, with or without additional active compounds or principles, and such unit dosage forms may contain any suitable effective amount of the active ingredient commensurate with the intended daily dosage range to be employed.
Compositions of this invention may also be liquid formulations including, but not limited to, aqueous or oily suspensions, solutions, emulsions and elixirs. Liquid forms suitable for topical use may include a suitable aqueous or non-aqueous vehicle with buffers, suspending and dispensing agents, colorants, flavors and the like.
The compositions may also be formulated as a dry product for reconstitution with water or other suitable vehicle before use. Such liquid preparations may contain additives including, but not limited to, suspending agents, emulsifying agents, non-aqueous vehicles and preservatives. Suspending agent include, but are not limited to, sorbitol syrup, methyl cellulose, gelatin, hydroxyethylcellulose, carboxymethyl cellulose, aluminum stearate gel, and hydrogenated edible fats. Emulsifying agents include, but are not limited to, lecithin, sorbitan monooleate, and acacia. Nonaqueous vehicles include, but are not limited to, edible oils, almond oil, fractionated coconut oil, oily esters, propylene glycol, and ethyl alcohol. Preservatives include, but are not limited to, methyl or propyl p-hydroxybenzoate and sorbic acid. Further materials as well as processing techniques and the like are set out in Part 5 of Remington's Pharmaceutical Sciences, 20th Edition, 2000, Marck Publishing Company, Easton, Pennsylvania, which is incorporated herein by reference.
Vehicles can be formulated with liquid or solid emollients, solvents, thickeners, humectants and/or powders. Emollients include stearyl alcohol, mink oil, cetyl alcohol, oleyl alcohol, isopropyl laurate, polyethylene glycol, petroleum jelly, palmitic acid, oleic acid, and myristyl myristate. Solvents include ethyl alcohol, isopropanol, acetone, diethylene glycol, ethylene glycol, dimethyl sulfoxide, and dimethyl formamide
Compositions of this invention may also be formulated transdermal formulations comprising aqueous or non-aqueous vehicles including, but not limited to, creams, ointments, lotions, pastes, medicated plaster, patch, or membrane.
Typical topical compositions include those pharmaceutical forms in which can be applied externally by direct contact with the surface to be treated. Conventional pharmaceutical forms for this purpose include ointments, waxes, lotions, pastes, jellies, sprays, aerosols, and the like in aqueous or non-aqueous formulations. The term "ointment" embraces formulations (including creams) having oleaginous, absorption, water-soluble and emulsion-type bases, e.g., petrolatum, lanolin, polyethylene glycols, as well as mixtures of these.
Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water.
Compositions of this invention may also be formulated as a depot preparation, which may be administered by subcutaneous injection or implantation. The compositions may be formulated with suitable polymeric or hydrophobic materials (as an emulsion in an acceptable oil, for example), ion exchange resins, or as sparingly soluble derivatives (as a sparingly soluble salt, for example).
The composition also can be formulated to provide a reservoir within or on the surface of the skin to provide for a continual slow release of the mTOR modulator. The composition also may be formulated to evaporate slowly from the skin, allowing the mTOR modulator extra time to penetrate the skin.
Compositions of this invention may also be formulated as a liposome preparation. The liposome preparation can comprise liposomes which penetrate the cells of interest or the *stratum corneum,* and fuse with the cell membrane, resulting in delivery of the contents of the liposome into the cell. Other suitable formulations can employ niosomes. Niosomes are lipid vesicles similar to liposomes, with membranes consisting largely of non-ionic lipids, some forms of which are effective for transporting compounds across the stratum corneum.
The compounds of this invention can also be administered in sustained release forms or from sustained release drug delivery systems. A description of representative sustained release materials can also be found in the incorporated materials in *Remington's Pharmaceutical Sciences.*
The invention is also related to dermatological or cosmetic compositions for topical treatment for the stimulation of hair growth which comprise an effective hair growth stimulating amount of one or more mTOR modulator such as a mTOR inhibitor like rapamicyn or an analog thereof as defined above and a dermatologically compatible carrier. Effective amounts of the mTOR modulator will vary analogues on the derivative employed, frequency of application and desired result, but will generally range from about 0.0000001 to about 50% by weight of the dermatological or cosmetic composition; preferably from about 0.00001 to about 5% by weight; and most preferably from about 0.0001 to about 0.1 % by weight. Representative compositions may thus comprise from about 0.001 to about 50 µg of the analogues in about 1 to about 100 µg of total dermatological composition, more preferably from about 0.01 to about 5 µg in about 10 to about 50 µg of the composition.
In forming compositions for topical administration, the compounds of the present invention are generally formulated as between about 0.00003 to about 3 percent by weight (wt %) solutions in water at a pH between 4.5 to 8.0. The compounds are preferably formulated as between about 0.0003 to about 0.3 wt% and, most preferably, between about 0.003 and about 0.03 wt%. While the precise regimen is left to the discretion of the clinician, it is recommended that the resulting solution be topically applied by spray, roll-on or dropper and massaged into the affected area, for example the scalp, once a day.
Other ingredients which may be desirable to use in the dermatological or cosmetic preparations of the present invention include preservatives, co-solvents and viscosity building agents.
The concentration of the mTOR modulator in the composition may be varied over a wide range up to a saturated solution, preferably from 0.1% to 30% by weight or even more.

The effective amounts may range, for example, from 0.1 ng to 1 mg or more per square centimeter of skin.

### Antimicrobial Preservatives:

Dermatological products are typically packaged in multidose form, which generally require the addition of preservatives to prevent microbial contamination during use. Suitable preservatives include: benzalkonium chloride, thimerosal, chlorobutanol, methyl paraben, propyl paraben, phenylethyl alcohol, edetate disodium, sorbic acid, ONAMERMO, or other agents known to those skilled in the art. Such preservatives are typically employed at a concentration between about 0.001 % and about 1.0% by weight.

### Co-Solvents:

Surfactants and appropriate co-solvent can be added to the in composition according to the invention. Such co-solvents include : Polysorbate 20, 60 and 80; Pluronic F-68, F-84 and P-103; Tyloxapol; Cremophor EL; sodium dodecyl sulfate; glycerol; PEG 400; propylene glycol; cyclodextrins; or other agents known to those skilled in the art. Such co-solvents are typically employed at a concentration between about 0.01 % and about 2% by weight.

### Viscosity Agents:

Viscosity greater than that of simple aqueous solutions may be desirable to increase skin absorption of the active compound, to decrease variability in dispensing the topical formulations, to decrease physical separation of components of a suspension or emulsion of formulation and/or otherwise to improve the topical formulation. Such viscosity building agents include, for example, polyvinyl alcohol, polyvinyl pyrrolidone, methyl cellulose, hydroxy propyl methylcellulose, hydroxyethyl cellulose, carboxymethyl cellulose, hydroxy propyl cellulose or other agents known to those skilled in the art. Such agents are typically employed at a concentration between about 0.01 % and about 2% by weight.

### Mode of administration

Topical use of a therapeutically active amount of the compositions of the present invention is defined as an amount effective, at dosages and for periods of time necessary to achieve the desired result.

A dosage regime may be adjusted to provide the optimum therapeutic response. For example, several divided doses may be administered daily, a single dose may be administered daily, weekly, monthly or at longer intervals, or the dose may be proportionally reduced as indicated by the exigencies of the therapeutic or cosmetic situation.
Compositions of this invention may be administered in any manner including, but not limited to, transdermal or topical administration, or combinations thereof. The compositions of this invention may also be administered in the form of an implant, which allows slow release of the compositions. In a preferred embodiment, a mTOR modulator according to the invention is administered topically.
This invention is further illustrated by the following examples that are not intended to limit the scope of the invention in any way.
The dosage administered, as single or multiple doses, to an individual will vary depending upon a variety of factors, including pharmacokinetic properties, patient conditions and characteristics (sex, age, body weight, health, size), extent of symptoms, concurrent treatments, frequency of treatment and the effect desired.

### Combination

According to the invention, a mTOR modulator according to the invention can be administered alone or in combination with a co-agent useful to treat such hair growth disorders, including corticosteroids, calcineurin inhibitors, topical minoxidil, and oral finasteride.
The invention encompasses the administration of mTOR modulator of the invention wherein the mTOR modulator is administered to an individual prior to, simultaneously or sequentially with other therapeutic regimens or co-agents useful in the treatment of to treat such hair growth disorders, in a therapeutically effective amount.
The combination therapy is useful for treating pathological conditions or disorders resulting in hair loss. The term "in combination" in this context means that the mTOR modulator such as rapamycin composition and a second therapeutic composition are given either simultaneously or sequentially. In one aspect, if given sequentially, at the onset of administration of the second compound, the first of the two compounds may still be detectable at effective concentrations at the site of treatment.
The mTOR modulator according to the invention that are administered simultaneously with said co-agents can be administered in the same or different compositions and in the same or different routes of administration.
For example, the combination therapy may include at least one mTOR modulator such as rapamycin composition co-formulated with and/or co-administered with, at least one additional therapeutic agent for stimulating hair growth. The additional agents may include at least one of the following, administered either topically or by injection: (1) corticosteroids, can include a class of natural and synthetic compounds, such as prednisone, dexamethasone, or hydrocortisone; (2) calcineurin inhibitors known to have immunosuppressive activity, such as cyclosporin A, pimecrolimus or tacrolimus; (3) minoxidil or (4) finasteride. Such combination therapies may advantageously utilize lower dosages of the administered therapeutic agents, thus avoiding possible toxicities or complications associated with the various monotherapies alone.

The mTOR modulator may be incorporated with the at least one additional therapeutic agent for stimulating hair growth such that they can both be applied in a single application. Alternatively, the mTOR modulator and at least one additional therapeutic agent for stimulating hair growth can be applied separately to the treatment area without departing from the improved benefit of this invention.
For example, a therapeutically active amount of at least one mTOR modulator, a corticosteroid, a calcineurin inhibitor, minoxidil, or finasteride may vary according to factors such as the disease state, age, sex, and weight of the individual, and the ability of the compound to elicit a desired response in the individual.

### Patients

In an embodiment, patients according to the invention are patients suffering from hair loss.
In another embodiment, patients according to the invention are patients suffering from hair growth deficiencies.

In another embodiment, patients according to the invention are patients suffering from undesired hair growth such as hirsutism.

### Use according to the invention

The nucleic acid according to the invention may be used in an assay according to the invention.In another aspect, the nucleic acid according to the invention may be used in an assay for the identification of mTOR modulators.
The present invention further relates to methods of controlling stem cells growth *in vitro* such as keratinocyte stem cells, foetal stem cells, adult somatic stem cells, non-human embryonic stem cells, hematopoietic cells or gut stem cells and spermatogonia cells.
The nucleic acid according to the invention may be used in a method of preparation of topical application.
The present invention further relates to methods of controlling hair growth such as stimulating hair growth and to pharmaceutical compositions that stimulate hair growth.
The present invention provides methods for treating hair loss disorders by topical administration of mTOR modulator such as rapamycin compositions. The invention further provides methods of treating hair loss disorders by administration of mTOR modulator such as rapamycin compositions in combination with co-agents.
Some aspects of the invention provide different pharmaceutical formulations of mTOR modulator such as rapamycin compositions to facilitate different routes of administration. Other aspects of the invention provide different dosage ranges or treatment regimens to treat a wide range of hair loss disorders. Exemplary hair loss disorders that may be treated with the compositions of the invention include alopecia areata, alopecia totalis, alopecia universalis, androgenic alopecia, telogen effluvium, anagen effluvium, and chemotherapy-induced alopecia.
Topical administration of the compositions of the invention as described herein may be as a therapeutically effective formulation containing a therapeutically active amount of at least one mTOR modulator such as rapamycin alone or in combination with any other therapeutic composition or molecule.

This invention relates to methods for treating disorders such as hair loss, pattern hair loss (alopecia), in particular. It also relates to enhance growth, fullness, thickness, and overall quality of scalp hair. Even more particularly, the invention relates to treatment of these conditions in females.
The invention relates to compositions which are useful in the stimulation of hair follicles, with a resulting benefit of treating hair growth disorders, such as alopecia.
Stimulation of hair growth is demonstrated when, for example, the rate of hair growth is increased, the number of hair is increased reduced, the subject perceives more hair on the treated site, or quantitatively, when the hair mass is increased, the hair resistance and texture is improved.
In another aspect, the invention relates to reducing hair growth in mammals, particularly for cosmetic purposes.
In one aspect, the invention provides a method (typically a cosmetic method) of reducing unwanted mammalian (preferably human) hair growth by applying to the skin a mTOR modulator such as rapamycin or analogs thereof in an amount effective to reduce hair growth. The unwanted hair growth may be undesirable from a cosmetic standpoint. Typically, in practicing the aforementioned methods, the mTOR modulator will be included in a topical composition along with a dermatologically or cosmetically acceptable vehicle. Accordingly, the present invention also relates to topical compositions comprising a dermatologically or cosmetically acceptable vehicle and a mTOR modulator.
In addition, the present invention relates to the use of mTOR modulator for the manufacture of a therapeutic topical composition for reducing hair growth.
The composition should be applied topically to a selected area of the body from which it is desired to reduce hair growth. For example, the composition can be applied to the face, particularly to the beard area of the face, i.e., the cheek, neck, upper lip, and chin. The composition also may be used as an adjunct to other methods of hair removal including shaving, waxing, mechanical epilation, chemical depilation, electrolysis and laser-assisted hair removal. Other actions that make their concept appearance are concurrent skin benefits in addition to hair reduction. The composition can also be applied to the legs, arms, torso or armpits. The composition is suitable, for example, for reducing the growth of unwanted hair in women. In humans, the composition should be applied once or twice a day, or even more frequently, to achieve a perceived reduction in hair growth. Reduction in hair growth is demonstrated when, for example, the rate of hair growth is slowed, the need for removal is reduced, the subject perceives less hair on the treated site, or quantitatively, when the weight of hair removed (i.e., hair mass) is reduced.
The disclosed nucleic acid sequences according to the invention and combinations thereof may be used as probes or primers.
Within the context of this invention, the beneficial effect of compositions and use according to the invention includes but is not limited to an attenuation, reduction, decrease or diminishing of the pathological development after onset of the disease.

In one embodiment, the invention provides an isolated nucleic acid molecule characterized by a sequence having at least 80% identity or homology (such as at least 85%, at least 90%, at least 95%, at least 98%) with a sequence of nucleic acid selected from the group consisting of: SEQ ID NO: 1; SEQ ID NO: 2; SEQ ID NO: 3; SEQ ID NO: 4; SEQ ID NO: 5; SEQ ID NO: 6, SEQ ID NO: 7 and the nucleic acid of their complement.

In a further embodiment, the invention provides an isolated nucleic acid molecule characterized by a sequence having at least 80% identity or homology (such as at least 85%, at least 90%, at least 95%, at least 98%) with a sequence of nucleic acid selected from the group consisting of: SEQ ID NO: 1; SEQ ID NO: 2; SEQ ID NO: 3; SEQ ID NO: 4; SEQ ID NO: 5, SEQ ID NO: 6 and the nucleic acid sequence of their complement.

In a further embodiment, the invention provides an isolated nucleic acid molecule characterized by a sequence having at least 80% identity or homology (such as at least 85%, at least 90%, at least 95%, at least 98%) with a sequence of nucleic acid selected from the group consisting of: SEQ ID NO: 1; SEQ ID NO: 2; SEQ ID NO: 3; SEQ ID NO: 4; SEQ ID NO: 5 and SEQ ID NO: 6.

In a further embodiment, the invention provides an isolated nucleic acid according to the invention selected from the group consisting of: SEQ ID NO: 8; SEQ ID NO: 9; SEQ ID: 10; SEQ ID NO: 11; SEQ ID NO: 12; SEQ ID NO: 13 and SEQ ID NO: 14.

In a further embodiment, the invention provides an isolated nucleic acid according to the invention selected from the group consisting of: SEQ ID NO: 8; SEQ ID NO: 9; SEQ ID: 10; SEQ ID NO: 11; SEQ ID NO: 12 and SEQ ID NO: 13.

In a further embodiment, the invention provides an isolated nucleic acid molecule according to the invention molecule selected from the group consisting of: SEQ ID NO: 1; SEQ ID NO: 2; SEQ ID NO: 3; SEQ ID NO: 4; SEQ ID NO: 5; SEQ ID NO: 6 and SEQ ID NO: 7.

In a further embodiment, the invention provides an isolated nucleic acid molecule according to the invention molecule selected from the group consisting of: SEQ ID NO: 1; SEQ ID NO: 2; SEQ ID NO: 3; SEQ ID NO: 4; SEQ ID NO: 5 and SEQ ID NO: 6.

In a further embodiment, the invention provides an isolated nucleic acid molecule characterized by a sequence which is a complement of a nucleic acid molecule selected from the group consisting of: SEQ ID NO: 1; SEQ ID NO: 2; SEQ ID NO: 3; SEQ ID NO: 4; SEQ ID NO: 5; SEQ ID NO: 6 and SEQ ID NO: 7.

In a further embodiment, the invention provides an isolated nucleic acid molecule characterized by a sequence which is a complement of a nucleic acid molecule selected from the group consisting of: SEQ ID NO: 1; SEQ ID NO: 2; SEQ ID NO: 3; SEQ ID NO: 4; SEQ ID NO: 5 and SEQ ID NO: 6.

In a further embodiment, the invention provides an isolated nucleic acid molecule characterized by a sequence which is a complement of a nucleic acid molecule selected from the group consisting of: SEQ ID NO: 8; SEQ ID NO: 9; SEQ ID: 10; SEQ ID NO: 11; SEQ ID NO: 12; SEQ ID NO: 13 and SEQ ID NO: 14.

In a further embodiment, the invention provides an isolated nucleic acid molecule characterized by a sequence which is a complement of a nucleic acid molecule selected from the group consisting of: SEQ ID NO: 8; SEQ ID NO: 9; SEQ ID: 10; SEQ ID NO: 11; SEQ ID NO: 12 and SEQ ID NO: 13.

In a further embodiment, the invention provides an isolated nucleic acid according to the invention wherein the nucleic acid binds to at least one mTOR protein or fragment thereof.

In another embodiment, the invention relates to a nucleic acid molecule characterized by a sequence which is a complement of a nucleic acid having according to the invention.

In another embodiment, the invention provides an isolated nucleic acid molecule having a sequence comprising at least two nucleic acid molecules characterized by a sequence having at least 80% identity or homology (such as at least 85%, at least 90%, at least 95%, at least 98%) with a sequence of nucleic acid selected from the group consisting of: SEQ ID NO: 1; SEQ ID NO: 2; SEQ ID NO: 3; SEQ ID NO: 4; SEQ ID NO: 5, SEQ ID NO: 6 and the nucleic acid sequence of their complement.

In a further embodiment, the invention provides an isolated nucleic acid molecule having a sequence comprising at least two nucleic acid molecules selected from the group consisting of: SEQ ID NO: 8; SEQ ID NO: 9; SEQ ID: 10; SEQ ID NO: 11; SEQ ID NO: 12 and SEQ ID NO: 13.

In a further embodiment, the invention provides an isolated nucleic acid molecule having a sequence comprising at least two nucleic acid molecules selected from the group consisting of: SEQ ID NO: 1; SEQ ID NO: 2; SEQ ID NO: 3; SEQ ID NO: 4; SEQ ID NO: 5; and SEQ ID NO: 6.

In another embodiment, the invention provides an isolated nucleic acid molecule having a sequence comprising at least two nucleic acid molecules according to the invention and further comprising at least one nucleic acid molecule having at least 80% identity or homology (such as at least 85%, at least 90%, at least 95%, at least 98%) with the nucleic acid sequence of SEQ ID NO: 7 or with the nucleic acid sequence of its complement.

In a further embodiment, the invention provides an isolated nucleic acid molecule having a sequence comprising at least two nucleic acid molecules according to the invention and further comprising at least one nucleic acid molecule of SEQ ID NO: 14.

In a further embodiment, the invention provides an isolated nucleic acid molecule having a sequence comprising at least two nucleic acid molecules according to the invention and further comprising at least one nucleic acid molecule of SEQ ID NO: 7.

In a further embodiment, the invention provides an isolated nucleic acid molecule having a sequence comprising at least two nucleic acid molecules according to the invention and wherein the nucleic acid molecule is selected from the group consisting of: SEQ ID NO: 15; SEQ ID NO: 16; SEQ ID NO: 17; SEQ ID NO: 18; SEQ ID NO: 19; and SEQ ID NO: 21.

In a further embodiment, the invention provides an isolated nucleic acid molecule having a sequence comprising at least two nucleic acid molecules according to the invention and wherein the nucleic acid molecule is SEQ ID NO: 19.

In another embodiment, the invention provides a purified and isolated DNA molecule comprising a polynucleotide sequence according to the invention and wherein the nucleic acid sequence is transcriptionally linked to a promoter.

In another embodiment, the invention provides an isolated nucleic acid molecule comprising the nucleic acid molecule according to the invention and a nucleic acid sequence conferring the activity of a reporter gene.

In another embodiment, the invention provides an expression vector comprising the nucleic acid molecule according to the invention.

In a further embodiment, the invention provides a recombinant expression vector comprising a nucleic acid molecule according to the invention, wherein the vector optionally comprises an expression control sequence, allowing expression in prokaryotic or eukaryotic host cells of the encoded polypeptide, operably linked to the nucleic acid molecule.

In another embodiment, the invention provides a host cell (e.g. prokaryotic or eukaryotic) transfected or transformed with an expression vector according the invention or a nucleic acid according to the invention.

In another embodiment, the invention provides a process for producing cells capable of expressing a polypeptide according to the invention, comprising genetically engineering cells with a vector or a nucleic acid according to the invention

In another embodiment, the invention provides a process for the preparation of a transgenic host comprising the steps of transfecting a host cell with the nucleic acid molecule according to the invention or with a vector according to the invention.

In another embodiment, the invention provides a kit comprising at least one nucleic acid molecule according to the invention. The kit according to the invention may be used in an assay of the invention or in a screening assay for the identification of modulators of mTOR.

In another embodiment, the invention provides a process for the identification of modulators of the mTOR signaling pathway comprising the steps of:
(a) Providing a nucleic acid molecule according to the invention;
(b) Providing a cell or plurality of cells in a sample;
(c) Transfecting cell or plurality of cells with the nucleic acid provided in (a);
(d) Incubating said cell, or plurality of cells, with at least one putative modulator agent;
(e) Measuring the transcription level of the nucleic acid provided in (a);
(f) Comparing the transcription level measured in (e) or with the transcription level of the nucleic acid provided in (a) in the absence of putative modulator agent;
(g) Selecting the modulating compound(s).

In a further embodiment, the invention provides a process for the identification of modulators of the mTOR signaling pathway according to the invention wherein nucleic acid molecule provided in step (a) is a nucleic acid molecule having a sequence comprising at least two nucleic acid molecules according to the invention and further comprising at least one nucleic acid molecule having at least 80% identity or homology (such as at least 85%, at least 90%, at least 95%, at least 98%) with the nucleic acid sequence of SEQ ID NO: 7 or the nucleic acid sequence of its complement.

In a further embodiment, the invention provides a process for the identification of modulators of the mTOR signaling pathway according to the invention wherein nucleic acid molecule provided in step (a) is selected from the group consisting of: SEQ ID NO: 15; SEQ ID NO: 16; SEQ ID NO: 17; SEQ ID NO: 18; SEQ ID NO: 19; and SEQ ID NO: 21.

In a further embodiment, the invention provides a process for the identification of modulators of the mTOR signaling pathway according to the invention wherein nucleic acid molecule provided in step (a) is SEQ ID NO: 19.

In a further embodiment, the invention provides a process for the identification of modulators of the mTOR signaling pathway according to the invention wherein the cell or the plurality of cells provided under step (b) is selected from the group of stem cells such as keratinocyte stem cells, foetal stem cells, adult somatic stem cells, non-human embryonic stem cells, hematopoietic or gut stem cells and spermatogonia cells.

In a further embodiment, the invention provides a process for the identification of modulators of the mTOR signaling pathway according to the invention wherein the cell or the plurality of cells provided under step (b) is a stem cell or a plurality of stem cells such as keratinocyte stem cells.

In another further embodiment, the invention provides a process for the identification of modulators of the mTOR signaling pathway according to the invention wherein the process comprising a further step (h) wherein the cell or the plurality of cells is subjected to a stress such as variable microenvironment stress which includes thermal stress, pH variations, nutrients and oxygen availability, UV radiation, shear stress and energy stress.

In another embodiment, the invention provides a use of a mTOR signaling pathway modulator for the preparation of a composition for the control of hair growth.

In a further embodiment, the invention provides a use of a mTOR signaling pathway modulator for the preparation of a composition for the control of hair growth wherein the composition is a pharmaceutical composition.

In a further embodiment, the invention provides a use of a mTOR signaling pathway modulator for the preparation of a composition for the control of hair growth wherein the composition is a cosmetic composition.

In a further embodiment, the invention provides a use of a mTOR signaling pathway modulator for the preparation of a composition for the control of hair growth wherein the control of hair growth is selected from the treatment of hair loss such as alopaecia and the treatment of hair growth disorders.

In another embodiment, the invention provides a use of a mTOR signaling pathway modulator for the preparation of a composition for the control of stem cells growth such as keratinocyte stem cells, foetal stem cells, adult somatic stem cells, non-human embryonic stem cells, hematopoietic cells or gut stem cells and spermatogonia cells.

In a further embodiment, the invention provides a use of a mTOR signaling pathway modulator for the preparation of a composition for the control of keratinocyte stem cells growth.

In another embodiment, the invention provides a use of a mTOR modulator for the manufacture of a medicament for the treatment of hair growth disorders comprising conditions or diseases such as hair loss, alopecia conditions wherein the mTOR modulator is to be administered in combination with a co-agent that useful in the treatment of hair growth disorders such as corticosteroids such as prednisone, dexamethasone, or hydrocortisone; calcineurin inhibitors such as cyclosporin A, pimecrolimus or tacrolimus; minoxidil or finasteride. In a particular embodiment, the mTOR modulator and the co-agent are used simultaneously or sequentially.

In another embodiment, the invention provides a method of treating of a disease or a condition comprising the topical application of a therapeutically effective amount of a mTOR modulator in a mammal in need thereof and wherein the disease or condition is a hair growth disorder comprising hair loss and alopecia. In a further embodiment, the mammal is human. In another further embodiment, the human suffers from a hair growth disorder such as hair loss and alopecia.

The invention relates to methods of promoting hair growth in a mammal comprising the topical application to the mammal a mTOR modulator in a effective amount. In a particular embodiment, the mammal in the methods of the present invention is a human.

In another embodiment, the invention provides a method of antagonizing hair growth in a mammal, comprising the topical application to the mammal a mTOR modulator in a effective amount such that the mTOR modulator blocks the growth of hairs or reduce the amount and/or rate of growth of hairs. More particularly, the mTOR modulator used in this method is rapamycin or everolimus.

In another embodiment, the invention provides a process for the preparation of epithelium grafts comprising the steps of:
(a) Providing a cell or plurality of keratinocyte stem cells in a sample;
(b) Incubating said cell, or plurality of cells, with at least one mTOR modulator;
(c) Harvesting the keratinocyte stem cells after incubation.

In a further embodiment, the invention provides a use or a process according to the invention wherein the mTOR modulator is a mTOR inhibitor.

In a further embodiment, the invention provides a use or a process according to the invention wherein the mTOR inhibitor is rapamycin or an analog thereof

In a further embodiment, the invention provides a use or a process according to the invention wherein the mTOR inhibitor is rapamycin.

In another embodiment, the invention provides a cosmetic composition comprising Rapamycin or an analogue thereof and a cosmetically acceptable carrier or vehicle for topical application.

In a further embodiment, the invention provides a cosmetic composition comprising Rapamycin or an analogue thereof and a cosmetically acceptable carrier or vehicle for topical application, in particular a cosmetic composition comprising at least 0.1 ng of rapamycin or of an analogue thereof, such as for example 0.1 ng-1 mg of Rapamycin or of an analogue thereof

References cited herein are hereby incorporated by reference in their entirety. The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description and accompanying drawings. Such modifications are intended to fall within the scope of the appended claims.
The invention having been described, the following examples are presented by way of illustration, and not limitation.

### Examples

The following abbreviations refer respectively to the definitions below:
**mg** (milligram), **min** (minute), **mM** (millimolar), µ**g** (microgram), **ng** (nanogram), **nM** (nanomolar), rpm (rotation per minute), **ChIP** Chromatin immunoprecipitation, DTT (1,4-Dithio-D,L-threitol), **EDTA** (ethylenediaminetetraacetic acid), **EGTA** (Ethylene-bis(oxyethylenenitrilo)tetraacetic acid), **GAPDH** (Glyseraldehyde-3-phosphate dehydrogenase), HSP (Horseradish Peroxidase), **PBS** (phosphate-buffered saline), **SDS** (Sodium Dodecyl Sulfate).

### Example 1: Temperature sensitive expression of genes in human keratinocytes

Human keratinocytes used were from the foreskin of a newborn (YF29) *(*Brouard et al., 1999, J Cell Sci 112 (Pt 19), 3343-3352*.)*, the thigh of a 78 year old female *(*Barrandon and Green, 1987a, Cell 50, 1131-1137*)* or a scalp hair follicle of a one year old male *(*Rochat et al., 1994, Cell 76, 1063-1073*).* Frozen cells were thawed and cultivated on a feeder layer of irradiated 3T3-J2 cells in Dulbecco Modified Eagle Medium F12 (3:1 v/v) supplemented as described *(*Oshima et al., 2001, Cell 104, 233-2*).*
YF29 cells were cultivated in presence of 100 nM of rapamycin (from Calbiochem) for a minimum of 4 hours and a maximum of 7 days and the expression of some genes such as CXCL14 (accession number: NM_004887 of SEQ ID NO: 22) and DNAJB5, accession number: NM_012266 of SEQ ID NO: 25 and analyzed by quantitative PCR with the following list of primers:
The cDNA of CXCL14 (SEQ ID NO: 22) was used as a template for PCR amplification with the primers 5'-GAAGCCAAAGTACCCG-3' of SEQ ID NO: 23 and 5'-TTCTTCGTAGACCCTGC -3 of SEQ ID NO: 24 respectively.
The cDNA of DNAJB5 (SEQ ID NO: 25) was used as a template for PCR amplification with the primers 5'-CAACGTGCTCTACAGT-3' of SEQ ID NO: 26 and 5'-GGAGTCTCTTCACGGT-3 of SEQ ID NO: 27, respectively.
The primers used for amplifying GAPDH were SEQ ID NO: 28 (5'-GAAGGTGAAGGTCGGAGT-3') and SEQ ID NO: 29 (5'-GAAGATGGTGATGGGATTTC-3').
   cDNAs were synthesized by adding 100 ng of random primers (Promega), 5 µg total RNA and dNTP Mix, heat mixture to 65°C for 5 min and incubate on ice. Then, add first-strand buffer, DTT, RNase inhibitor and SuperScript III (Invitrogen), incubate at 25°C for 5 min, at 60°C for 1 hour and inactivate the reaction by heating at 70°C for 15 min. The cDNA is used as a template for quantitative PCR. cDNAs were adjusted to equal levels by PCR amplification with primers to 18S 5'-CGGCTACCACATCCAAGGAA-3' of SEQ ID NO: 30 and 5'-GCTGGAATTACCGCGGCT-3 of SEQ ID NO: 31, respectively and GAPDH. Primers were designed to ensure the uniqueness for each gene using LightCycler Probe Design 1.0 software and NCBI BLAST (http://www.ncbi.nlm.nih.gov/BLAST/).

Quantitative PCR was performed using LightCycler FastStart DNA Master SYBR Green I reagents, a LightCycler System piloted by a LightCycler 3.5 software (Roche Diagnostics). Differences between experimental samples and controls were calculated on the basis of the 2^{-DDCT} method as disclosed in *(*Nicolas et al., 2003, Nature genetics 33, 416-421*).*
A two-fold decrease in the expression of CXCL14 and DNAJB5 was constantly observed. Its suggests that keratinocyte stem cells could regulate gene expression in response to rapamycin treatment or a change in their microenvironment through mTOR signaling and that CXCL14 and DNAJB5 are temperature responsive genes.

### Example 2: Minimal sequence transcriptionally active interacting with mTOR

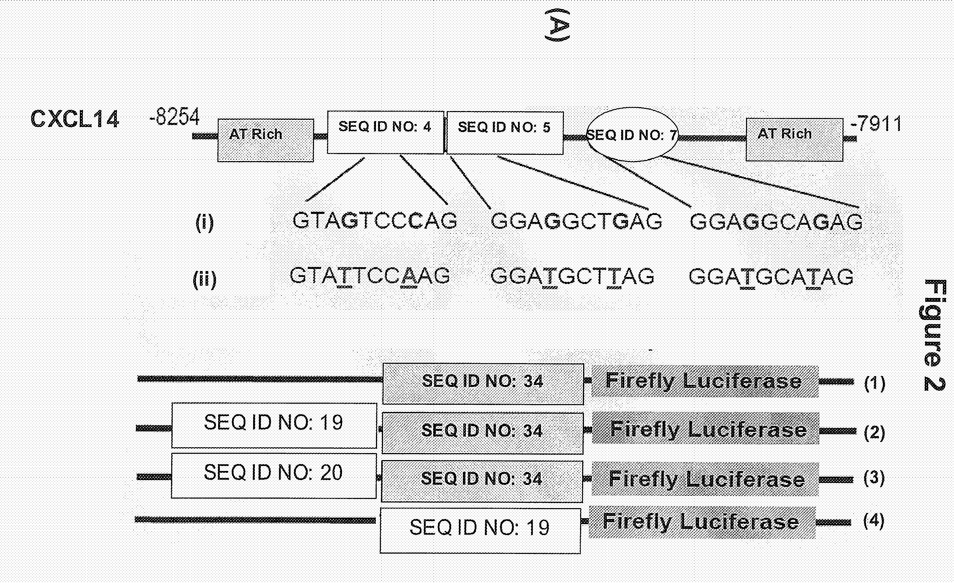

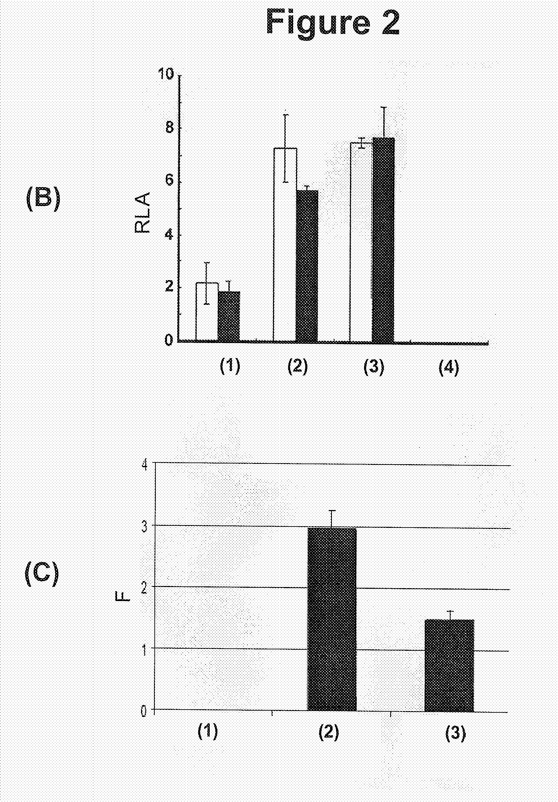

DNA fragments encoding a wild type (SEQ ID NO: 19) or a mutated (SEQ ID NO: 20) wherein 2 nucleotides in each motif of SEQ ID NO: 4, SEQ ID NO: 5 and SEQ ID NO: 7 are substituted (Figure 2A) of the human CXCL14 gene (NT_034772.5, SEQ ID NO: 32) (-8212 to -8020) were generated by PCR and cloned in a pGL3b vector (Promega) which is based on the pGL3b plasmid of SEQ ID NO: 33, upstream of a HPV16 promoter of SEQ ID NO: 34 (fragment 7154-7904 from HPV16 genome, K02718) known to be active in human keratinocytes *(*Bechtold et al., 2003, J Virol 77, 2021-2028*)* driving a firefly luciferase gene (Figure 2A).
Controls were pGL3b plasmids of SEQ ID NO: 33 in which a wild type sequence of SEQ ID NO: 19 was cloned without the HPV16 promoter of SEQ ID NO: 34 (**4**), a HPV16 promoter of SEQ ID NO: 34 was cloned alone (**1**), a SV40 promoter SEQ ID NO: 35 was driving a renilla luciferase gene in a pRL-SV40 vector (Promega) based on the plasmid of SEQ ID NO: 36.
YF29 cells prepared as described in Example 1 were cultivated at 36° C before they were transfected with a luciferase plasmid containing a sequence of SEQ ID NO: 19 positioned upstream of a HPV16 promoter of SEQ ID NO: 34 (**2**) and the above control plasmids (**1**) and (**4**) (Figure 2A). Transfected cells were then cultivated at 36° C or at 37° C for 24 hours.
All cloned DNA fragments were sequenced (Microsynth, Switzerland). YF29 cells were cultivated at 36° C for 24 hours onto an irradiated feeder layer of 3T3 cells as described in Rheinwald and Green, 1975, Cell 6, 331-343 before they were transfected with the above plasmids using a Fugene 6 transfection reagent (Roche). After an overnight incubation at 36°C, cells were either kept at 36°C or switched to 37°C for 24 hours. Cells were then scraped and lysed in reporter lysis buffer (Promega). Luciferase or renilla activity in the cell extracts was assayed using a specific kit (Promega); renilla luciferase served as an indicator of transfection efficiency, whereas firefly luciferase revealed the activity of the SEQ ID NO: 19 and/or HPV16 sequences. Relative luciferase activity was calculated by standardizing the firefly luciferase activity to that of the renilla (LUMAT LB 9507, Berthold Technologies, US). The values (means ± S.D.) were determined based on triplicate experiments. P values were obtained using the Student's t test.
Luciferase activity (Figure 2B) was significantly increased in cells transfected with a SEQ ID NO: 19-HPV16 plasmid (**2**) compared to cells transfected with control plasmids only containing either a HPV16 promoter of SEQ ID NO: 34 (**1**) or a SEQ ID NO: 19 sequence alone (**4**) (Figure 2B). Furthermore, there was a significant reduction in luciferase activity when cells were cultivated at 37°C versus 36°C, suggesting that SEQ ID NO: 19 was involved in the transcriptional response to a temperature change; importantly, this difference was abolished when cells were transfected with a plasmid containing a mutated sequence of SEQ ID NO: 19 (SEQ ID NO: 20) (**3**) of in each of the three motifs SEQ ID NO: 4, SEQ ID NO: 5 and SEQ ID NO: 7 (targeted substitution of 2 nucleotides in each motif) (Figure 2A).
Chromatin immunoprecipitation (ChIP) analysis using antibodies to mTOR as described in Example 3 below demonstrated that the cloned sequence of SEQ ID NO: 19 could associate with proteins involved in the regulation of transcription, namely through mTOR signaling (Figure 2C).
This indicates that sequence of SEQ ID NO: 19 is a minimal sequence that could be transcriptionally active and that was sufficient to confer temperature sensitivity to a responsive gene.

### Example 3: mTOR complexes association with sequences of the invention

To further investigate the nuclear role of mTOR, ChIP analyses were performed as described in Aparicio et al., 2004, In Current Protocols in Molecular Biology, F. Ausubel, ed. (Indianapolis, Wiley Publishing Inc.), pp. 21.23.21-21.23.23*)* using antibodies to proteins of the mTORC1 and mTORC2 cytoplasmic complexes, i.e. antibodies to mTOR (Sigma, T2949), RAPTOR (Abcam, Ltd, ab26264) and RICTOR (ab32892), respectively.

### mTOR immunostaining:

YF 29 keratinocyte stem cells prepared as described in Example 1 were treated for 3 days onto an irradiated feeder layer of 3T3 cells as described above and then treated with or without 100nM rapamycin for 4 hours. Cells were then immunostained with mTOR antibodies and Alexa Fluor 568 and examined by confocal microscopy: cells were washed twice with PBS and fixed in 10% formalin for 10 minutes before they were treated with 0.5% Triton X-100 and subsequently with 1% bovine serum albumin. Cells were then incubated with the rabbit polyclonal antibody to mTOR described above recognizing phosphorylated and non-phosphorylated forms for 2 hours, washed several times in PBS, and subsequently incubated with Alexa Fluor 568-conjugated goat anti-rabbit IgG (Dako) for 1 hour. After a PBS wash, cells were stained with Hoechst 33258 (Molecular probes), mounted in fluorescent mounting medium (Dako) and examined under a Leica SP2 invert confocal microscope. Negative controls used were either no primary antibody or the vehicle alone (no rapamycin).

### Nuclear mTOR:

YF29 cells were cultivated onto a feeder layer of irradiated 3T3 cells for 3 days without or with 100nM rapamycin before they were quickly scraped from the culture dish in a small volume of PBS. Cells were centrifuged and pellets were resuspended in 200 µl of lysis buffer A (10 mM Hepes pH 7.9, 10 mM KCI, 0.1 mM EDTA, 0.1 mM EGTA, 1 mM DTT) containing a cocktail of protease inhibitors (Roche). Lysates were then incubated on ice for 15 minutes before 12.5 µl of 10 % Triton X-100 was added. The diluted cell lysates were then centrifuged at 14.000 rpm for 30 seconds. Supernatants were collected as the cytoplasmic fractions, whereas pellets containing the cell nuclei were resuspended by vigorous pipeting with 50 µl of lysis buffer B (20 mM Hepes pH 7.9, 400 mM NaCl, 1 mM EDTA, 1 mM EGTA, 1 mM DTT, and protease inhibitors). The lysed nuclei were then incubated on ice for 15 minutes before they were centrifuged at 14.000 rpm for 15 minutes. Supernatants were then collected as the nuclear fractions. Ten µg of proteins of each cytoplamic and nuclear fractions were dissolved in SDS sample buffer and separated by 7% SDS-PAGE and transferred to nitrocellulose membranes (Schleicher & Schuell). After blocking with 5% skim milk in PBS, membranes were immunoblotted overnight at 4° C with the following primary antibodies: a rabbit anti-mTOR antibody (Sigma, ref. T2949), mouse anti-lamin A antibody (Abcam, ref. ab8980), or mouse anti-α-tubulin antibody (Sigma, ref. T5168). Membranes were then washed with 0.05% Tween-20 in PBS, and incubated with horseradish peroxidase (HRP)-conjugated anti-rabbit or anti-mouse antibody (Jackson Immunoresearch, ref. 115-035-003 or 111-035-0035) for 1 hour at room temperature. They were then washed with 0.05% Tween-20 in PBS, treated with an enhanced chemiluminescence substrate for HRP detection (Pierce) for 5 minutes, and exposed to Kodak BioMAX MR films. The bands were scanned and their density was measured using Scion Image software (Scion Corporation).
The observed mTOR nuclear localization was clearly increased with rapamycin treatment about two fold). Western blot on cytoplasmic and nuclear fractions showed that mTOR was present both in the cytoplasm and the nucleus. mTOR and members of mTORC1 and mTORC2 (RICTOR) associated with SEQ ID NO: 18 present in the promoter of DUSP4 (NM_001394, -9999 to 3000) SEQ ID NO: 21, present in the promoter region of EGFR (NT_033968, -9999 to 3000 of SEQ ID NO: 37) and with SEQ ID NO: 19 present in the promoter region of CXCL14 (SEQ ID NO: 32).
It was observed by ChIP as described above that mTOR, RAPTOR and RICTOR as well as FKBP12 clearly associated with nucleotide sequences selected from the group consisting of SEQ ID NO: 1, SEQ ID NO:2, SEQ ID NO: 3, SEQ ID NO: 4, SEQ ID NO: 5 and SEQ ID NO: 6 but not to a control sequence (SEQ ID NO: 32) that contained several IFHL-like motifs different from the sequences of SEQ ID NO: 1 to 6 (e.g. a sequence located in the vicinity of the CXCL14 transcription start site).
This demonstrates that mTOR nuclear complexes include at least members of mTORC1 or mTORC2 cytoplasmic complexes and associate to at least two sequences motifs selected from the group consisting of SEQ ID NO: 1, SEQ ID NO: 2, SEQ ID NO: 3, SEQ ID NO: 4, SEQ ID NO: 5 and SEQ ID NO: 6 altogether with RAP1, ILF1 and HDAC2, the human orthologs of the yeast TOR transcriptional complex. It demonstrates that sequence patterns of SEQ ID NO: 1 to 6 are important since any IFHL-like motif is by itself incapable to associate with ILF 1 and RAP1.

### Example 4:Treatment of human keratinocyte stem cells with rapamycin

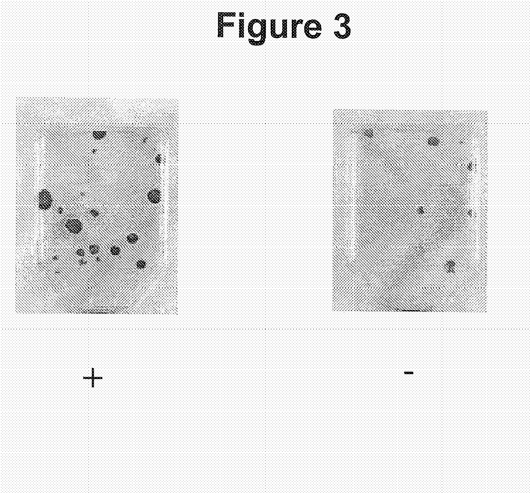

We cultivated YF29 human keratinocytes that were prepared as described in Example 1 with 100 nM rapamycin (Calbiochem) or its vehicle as described in the article. A classical plating efficiency was then done on these rapamycin-treated or control cells as described previously which allows to quantify the number of colonies formed starting from 100 seeded cells. The number of colonies is higher with rapamycin-treated cells and most importantly the colonies are larger (Figure 3). This result suggests that rapamycin might protect stem cells. YF29 cultivated in presence of 100 nM rapamycin present a modified morphology: the number of stratified or non growing colonies is clearly decreased and cells seem to be smaller.
Altogether, these results strongly suggest that a modulator of mTOR signaling pathway such as rapamycin could act on cell fate, migration, epithelial stratification and wound healing. Therefore, the *in vitro* increase of the number and/or quality of stem cells is a potential for improving the preparation of epithelium graft cultures, in particular for use on heavily burnt patients.

## Claims

1. An isolated nucleic acid molecule **characterized by** a sequence having at least 80% identity or homology with a sequence of nucleic acid selected from the group consisting of: SEQ ID NO: 1; SEQ ID NO: 2; SEQ ID NO: 3; SEQ ID NO: 4; SEQ ID NO: 5; SEQ ID NO: 6; SEQ ID NO: 7 and the nucleic acid sequence of their complement.

2. An isolated nucleic acid according to claim 1 selected from the group consisting of: SEQ ID NO: 8; SEQ ID NO: 9; SEQ ID: 10; SEQ ID NO: 11; SEQ ID NO: 12; SEQ ID NO: 13 and SEQ ID NO: 14.

3. An isolated nucleic acid according to claims 1 to 2 wherein consisting of a nucleotide selected from the group consisting of: SEQ ID NO: 1; SEQ ID NO: 2; SEQ ID NO: 3; SEQ ID NO: 4; SEQ ID NO: 5; SEQ ID NO: 6 and SEQ ID NO: 7.

4. An isolated nucleic acid according to any ones of claim 1 to 3 and wherein the nucleic acid binds to at least one mTOR protein or fragment thereof

5. An isolated nucleic acid molecule **characterized by** a sequence which is a complement of a nucleic acid according to any ones of claims 1 to 4.

6. An isolated nucleic acid molecule having a sequence comprising at least two nucleic acid molecules each **characterized by** a sequence having at least 80% identity or homology with a sequence of nucleic acid selected from the group consisting of: SEQ ID NO: 1; SEQ ID NO: 2; SEQ ID NO: 3; SEQ ID NO: 4; SEQ ID NO: 5; SEQ ID NO: 6 and the nucleic acid of their complement.

7. A nucleic acid molecule according to claim 6 and further comprising at least one nucleic acid molecule having at least 80% identity or homology with the nucleic acid sequence of SEQ ID NO: 7 or the nucleic acid of its complement.

8. An isolated nucleic acid molecule according to any ones of claims 6 or 7 selected from the group consisting of: SEQ ID NO: 15; SEQ ID NO: 16; SEQ ID NO: 17; SEQ ID NO: 18; SEQ ID NO: 19; and SEQ ID NO: 21.

9. A process for the identification of modulators of the mTOR signaling pathway comprising the steps of:
(a) Providing a nucleic acid molecule according to any ones of claims 6 to 8;
(b) Providing a cell or plurality of cells in a sample;
(c) Transfecting cell or plurality of cells with the nucleic acid provided in (a);
(d) Incubating said cell, or plurality of cells, with at least one putative modulator agent;
(e) Measuring the transcription level of the nucleic acid provided in (a);
(f) Comparing the transcription level measured in (e) or with the transcription level of the nucleic acid provided in (a) in the absence of putative modulator agent;
(g) Selecting the modulating compound(s).

10. A process according to claim 9 wherein the cell or the plurality of cells provided under step (b) is a stem cell or a plurality of stem cells.

11. A use of a mTOR signaling pathway modulator for the preparation of a composition for the control of hair growth.

12. A use of a mTOR signaling pathway modulator for the in vitro control of stem cells growth.

13. A use according to claims 11 or 12, wherein the mTOR modulator is rapamycin.

14. A use according to any ones of claims 12 to 13 wherein the stems cells are keratynocyte stem cells.

15. A process for the preparation of epithelium grafts comprising the steps of:
(a) Providing a cell or plurality of keratynocyte stem cells in a sample;
(b) Incubating said cell, or plurality of cells, with at least one mTOR modulator;
(c) Harvesting the keratynocyte stem cells after incubation.

16. A cosmetic composition comprising Rapamycin or an analogue thereof and a cosmetically acceptable carrier or vehicle for topical application.

17. A purified and isolated DNA molecule comprising a polynucleotide sequence according to any ones of claims 1 to 8 and wherein the nucleic acid sequence is transcriptionally linked to a promoter.

18. An isolated nucleic acid molecule comprising the nucleic acid molecule according any ones of claims 1 to 8 and a nucleic acid sequence conferring the activity of a reporter gene.

19. An expression vector comprising the nucleic acid molecule according to any ones of claims 1 to 8.

20. A recombinant expression vector comprising a nucleic acid molecule according to any ones of claims 1 to 8, wherein the vector optionally comprises an expression control sequence, allowing expression in prokaryotic or eukaryotic host cells of the encoded polypeptide, operably linked to the nucleic acid molecule.

21. A host cell transfected or transformed with an expression vector according to claims 19 or 20 or a nucleic acid according any claims 1 to 8.

22. A process for the preparation of a transgenic host comprising the steps of transfecting a host cell with the nucleic acid molecule according to any ones of claims 1 to 8 or with a vector according to claims 19 or 20.

23. A kit comprising at least one nucleic acid molecule according to any claims 1 to 8.


SEQUENCE LISTING

<110>  Ecole Polytechnique Fédérale de Lausanne (EPFL)

<120>  Target of rapamycin modulators of and use thereof

<130>  P-26-659-EPY

<160>  37

<170>  PatentIn version 3.3

<210>  1
<211>  20
<212>  DNA
<213>  Homo sapiens

<400>  1
tcccagcact ttgggaggcc                                                    20


<210>  2
<211>  20
<212>  DNA
<213>  Homo sapiens

<400>  2
agaccagcct ggccaacatg                                                    20


<210>  3
<211>  20
<212>  DNA
<213>  Homo sapiens

<400>  3
ctctactaaa aatacaaaaa                                                    20


<210>  4
<211>  20
<212>  DNA
<213>  Homo sapiens

<400>  4
gcctgtagtc ccagctactc                                                    20


<210>  5
<211>  20
<212>  DNA
<213>  Homo sapiens

<400>  5
ggaggctgag gcaggagaat                                                    20


<210>  6
<211>  20
<212>  DNA
<213>  Homo sapiens

<400>  6
ccactgcact ccagcctggg                                                    20


<210>  7
<211>  10
<212>  DNA

<213>  Artificial

<220>
<223>  Synthetic sequence


<220>
<221>  misc_feature
<222>  (7)..(7)
<223>  n is a, c, g, or t

<400>  7
ctaggcngaa                                                              10


<210>  8
<211>  20
<212>  DNA
<213>  Artificial

<220>
<223>  Synthetic sequence


<220>
<221>  misc_feature
<222>  (4)..(4)
<223>  is selected from c and t

<220>
<221>  misc_feature
<222>  (6)..(6)
<223>  is selected from g and a

<220>
<221>  misc_feature
<222>  (10)..(11)
<223>  is selected from t and c, preferably t

<220>
<221>  misc_feature
<222>  (12)..(12)
<223>  is selected from t and a, preferably t

<220>
<221>  misc_feature
<222>  (14)..(15)
<223>  is selected from g and c, preferably g

<220>
<221>  misc_feature
<222>  (16)..(16)
<223>  is selected from a, t, g and c

<220>
<221>  misc_feature
<222>  (17)..(17)
<223>  is selected from g and c, preferably g

<220>
<221>  misc_feature
<222>  (18)..(18)
<223>  is selected from g and a, preferably g

<220>
<221>  misc_feature
<222>  (19)..(19)
<223>  is selected from c and a, preferably c

<220>
<221>  misc_feature
<222>  (19)..(19)
<223>  is selected from c and a, preferably c

<220>
<221>  misc_feature
<222>  (20)..(20)
<223>  is selected from c and t

<400>  8
tccnancacn nngnnngnnn                                                   20


<210>  9
<211>  20
<212>  DNA
<213>  Artificial

<220>
<223>  Synthetic sequence


<220>
<221>  misc_feature
<222>  (5)..(5)
<223>  is selected from c and t, preferably c

<220>
<221>  misc_feature
<222>  (8)..(8)
<223>  is selected from c and g, preferably c

<220>
<221>  misc_feature
<222>  (10)..(10)
<223>  is selected from a, t, c and g

<220>
<221>  misc_feature
<222>  (11)..(11)
<223>  is selected from g and c, preferably g

<220>
<221>  misc_feature
<222>  (12)..(12)
<223>  is selected from g and a

<220>
<221>  misc_feature
<222>  (13)..(13)
<223>  is selected from c and g

<220>
<221>  misc_feature
<222>  (14)..(14)
<223>  is selected from c and t

<220>
<221>  misc_feature
<222>  (19)..(19)
<223>  is selected from t and c

<220>
<221>  misc_feature
<222>  (20)..(20)
<223>  is selected from g and a

<400>  9

agacnagncn nnnnaacann                                                20

<210>  10
<211>  20
<212>  DNA
<213>  Artificial

<220>
<223>  Synthetic sequence


<220>
<221>  misc_feature
<222>  (1)..(1)
<223>  is selected from c and t

<220>
<221>  misc_feature
<222>  (2)..(2)
<223>  is selected from t and a

<220>
<221>  misc_feature
<222>  (3)..(3)
<223>  is selected from c and a

<220>
<221>  misc_feature
<222>  (4)..(4)
<223>  is selected from t and a

<220>
<221>  misc_feature
<222>  (6)..(6)
<223>  is selected from c and a

<220>
<221>  misc_feature
<222>  (7)..(7)
<223>  is selected from t and a

<220>
<221>  misc_feature
<222>  (8)..(8)
<223>  is selected from a and t

<220>
<221>  misc_feature
<222>  (12)..(12)
<223>  is selected from a and t

<220>
<221>  misc_feature
<222>  (13)..(13)
<223>  is selected from t and a

<220>
<221>  misc_feature
<222>  (15)..(15)
<223>  is selected from c and a

<220>
<221>  misc_feature
<222>  (20)..(20)
<223>  is selected from a and t

<400>  10
nnnnannnaa annanaaaan                                                20

<210>  11
<211>  20
<212>  DNA
<213>  Artificial

<220>
<223>  Synthetic sequence


<220>
<221>  misc_feature
<222>  (1)..(1)
<223>  is selected from g and a

<220>
<221>  misc_feature
<222>  (5)..(5)
<223>  is selected from g and a

<220>
<221>  misc_feature
<222>  (9)..(9)
<223>  is selected from t and g

<220>
<221>  misc_feature
<222>  (11)..(11)
<223>  is selected from a, c, g and t

<220>
<221>  misc_feature
<222>  (12)..(12)
<223>  is selected from c and g

<220>
<221>  misc_feature
<222>  (14)..(14)
<223>  is selected from g and a, preferably g

<220>
<221>  misc_feature
<222>  (17)..(17)
<223>  is selected from a and g

<220>
<221>  misc_feature
<222>  (20)..(20)
<223>  is selected from c and t

<400>  11
ncctntagnc nnanctnctn                                                    20


<210>  12
<211>  20
<212>  DNA
<213>  Artificial

<220>
<223>  Synthetic sequence


<220>
<221>  misc_feature
<222>  (1)..(1)
<223>  is selected from g and c, preferably g

<220>
<221>  misc_feature
<222>  (2)..(2)
<223>  is selected from g and t, preferably g

<220>
<221>  misc_feature
<222>  (7)..(7)
<223>  is selected from a, c, g and t

<220>
<221>  misc_feature
<222>  (8)..(8)
<223>  is selected from g and c, preferably g

<220>
<221>  misc_feature
<222>  (10)..(10)
<223>  is selected from g and a, preferably g

<220>
<221>  misc_feature
<222>  (11)..(11)
<223>  is selected from g and a

<220>
<221>  misc_feature
<222>  (12)..(12)
<223>  is selected from c and t

<220>
<221>  misc_feature
<222>  (13)..(13)
<223>  is selected from a and g

<220>
<221>  misc_feature
<222>  (15)..(15)
<223>  is selected from g and a

<220>
<221>  misc_feature
<222>  (17)..(17)
<223>  is selected from g and a

<220>
<221>  misc_feature
<222>  (18)..(18)
<223>  is selected from a and g

<400>  12
nnaggcnnan nnngnannat                                                    20


<210>  13
<211>  20
<212>  DNA
<213>  Artificial

<220>
<223>  Synthetic sequence


<220>
<221>  misc_feature
<222>  (1)..(1)
<223>  is selected from c and t

<220>

<221>  misc_feature
<222>  (4)..(4)
<223>  is selected from c and t

<220>
<221>  misc_feature
<222>  (7)..(7)
<223>  is selected from c and t

<220>
<221>  misc_feature
<222>  (12)..(12)
<223>  is selected from c and t

<220>
<221>  misc_feature
<222>  (15)..(15)
<223>  is selected from c and g, preferably g

<220>
<221>  misc_feature
<222>  (16)..(16)
<223>  is selected from c and t

<220>
<221>  misc_feature
<222>  (17)..(17)
<223>  is selected from a, c, g and t

<220>
<221>  misc_feature
<222>  (18)..(18)
<223>  is selected from g and c, preferably g

<220>
<221>  misc_feature
<222>  (19)..(20)
<223>  is selected from g and a

<400>  13
ncantgnact cnagnnnnnn                                                    20


<210>  14
<211>  10
<212>  DNA
<213>  Artificial

<220>
<223>  Synthetic sequence


<220>
<221>  misc_feature
<222>  (1)..(1)
<223>  is selected from c and g

<220>
<221>  misc_feature
<222>  (2)..(2)
<223>  is selected from t and c

<220>
<221>  misc_feature
<222>  (3)..(3)
<223>  is selected from a and t

<220>
<221>  misc_feature

<222>  (5)..(5)
<223>  is selected from g and c

<220>
<221>  misc_feature
<222>  (6)..(6)
<223>  is selected from c and g

<220>
<221>  misc_feature
<222>  (7)..(7)
<223>  is selected from a, c, g and t

<220>
<221>  misc_feature
<222>  (8)..(8)
<223>  is selected from g and c, preferably g

<220>
<221>  misc_feature
<222>  (9)..(10)
<223>  is selected from a and g

<400>  14
   nnngnnnnnn                                                          10


<210>  15
<211>  395
<212>  DNA
<213>  Homo sapiens

<400>  15
atgacacaga gaaaaataaa attggagctc tatctcacac catatacaag gacgaactct      60

acatggatta aagacctaaa tgtggccggg tgcggtggct catgcctgta atcccagcac     120

tttgggaggc cgaggcaggc gaatcacttg aggccaggag ttcaagacca gcctggccaa     180

catggcaaaa acctgtctct actaaaaatg caaaaattag ccaggtgtgg tggcacacac     240

ctgtaatcct agctacgcag caggctgagg caggagaatt gcttgaaccc aggaggcgga     300

ggttgcagtg agccgagata gtgccattgc actccagcct gggcaacaag agcgaaactc     360

tgtcaaaaaa aaaaaaaaag aaagaaagaa agaaa                                395


<210>  16
<211>  383
<212>  DNA
<213>  Homo sapiens

<400>  16
aattgataaa actcatttgc aaattatatg agtgactacc ttaaagaacc aacagagtcg      60

ggcgcggtgg ctcacgcctg taatcccagc attttgggag gccgaggcgg gcggatcacg     120

aggtcaggag atggagacca tcctggctaa cacggtgaaa ccccatctct actaaaagca     180

caaaaaatta gccaagtgtg gtggcgggca cctgtagtcc cagctactca ggaggctaag     240

gcaggagaat ggtgtgaacc cgggaggtgg agcttgcggt gagccaagat tgtgccactg     300

cactccagcc tgggtgacag agcgagactc catctcaaaa aaaaaaaaaa aaagaacaaa     360

cagaatgaca aaaacaacta ata                                             383

<210>  17
<211>  395
<212>  DNA
<213>  Homo sapiens

<400>  17
aaataaccta gttttgtttt gtattgtttt gtttgtttgt cttttgagat ggagtctcgc      60

tgtctcgccc tgtccaggca ggagtgcagt ggcccaatct tgactcactg caacctctgc     120

ctctccgggt caagggattc ttttgcctca gcctcacgag tagctgggac tacaggcacc     180

caccatcatg cctgactaat ttttgtattt ttaatgttgg tcaggctggt ctccaactcc     240

tgacctcaag tgatccgcca gcctcccaaa gtgctggggg attacaggca tgagccacca     300

tccatgccca gcctccgagt tcatttttat cccgtttgga gaagtgtttc tttccaaaat     360

ttgtttctga aattttgaag acgttttatc aaaat                                395


<210>  18
<211>  428
<212>  DNA
<213>  Homo sapiens

<400>  18
ttagaaactt aatcttttat tattttttat ttgtatttat ttatttattt tgagatggag      60

ttttgctctt gtcgcccagg ctggagtgca gtgaggggat ctcagctcac tgcaacctct     120

acctcccggg ttcaagcgat tctcctgcct cagcctccag tgtagctggg attatgggtg     180

cgcgcccgtc atgctggcta attttgtatt tttagaagag acagggtttc accatgttga     240

ccaggctggt cttaaactcc tgacctcagg tgatctgccc acttcggtct tccaaagtgc     300

tgggattact agcgtgagcc acctcgcctg gccgatacat agccttttaa atcattaata     360

agtgctagag tatcaagcat agaaaacaaa aaagatctgt aacaaagtca ggacttaaaa     420

aaagaaat                                                              428


<210>  19
<211>  199
<212>  DNA
<213>  Homo sapiens

<400>  19
tacaagaaaa tttaaaaact agccaagggt agtggcatgt gcctgtagtc ccagctgctt      60

gggaggctga ggtgggagga tcacttgcgc ctgggaggca gaggttgcaa tgagctgaga     120

tcacaccact gcactccaac ctgagtgaca gaacaagacc ctgtctcaaa aaaacaaaac     180

aaaacaaaaa aaatacaat                                                  199


<210>  20
<211>  199
<212>  DNA
<213>  Homo sapiens

<400>  20
tacaagaaaa tttaaaaact agccaagggt agtggcatgt gcctgtattc caagctgctt      60

gggatgctta ggtgggagga tcacttgcgc ctgggatgca taggttgcaa tgagctgaga     120

tcacaccact gcactccaac ctgagtgaca gaacaagacc ctgtctcaaa aaaacaaaac 180

aaaacaaaaa aaatacaat 199

<210> 21
<211> 232
<212> DNA
<213> Homo sapiens

<400> 21
aacatagcaa ggcactatct atacaaacat aattttaaaa agtccaggca ttagctagga 60

ctgtgcctgt agtcccagct actcaggagg ctgagatgag aagatcaccg gagcctagaa 120

atttgaggct gcagtgggct gtgatcatgt cacttcactc ctgcctagaa gacagttaga 180

ccctgcctct aaaataaaca agcaaataaa taaaaagaaa ggaagaaaag aa 232

<210> 22
<211> 1968
<212> DNA
<213> Homo sapiens

<400> 22
aatgtgtgcg cgctgtggta tgggtgtgca agtgtgcgaa ggcggcgtgt tgtgtgagcg 60

agagggtagc ggatgtgtgt gtgcgtgtgc gcgcgtggct ccgggtgtgc gccgctgcga 120

tagcgggtcc tttcccgggg cgggcgacgg gcgggctggg aaggtctcct cccctcacca 180

cattgagaaa tctcagtgag tcaccgagtg gttctgcata ttaatgagct cgctcgctgc 240

gagggcagga gcggatttaa aagaggccag ggcgggcgga gggaggctgt ggagagagcg 300

cggagacaag cgcagagcgc agcgcacggc cacagacagc cctgggcatc caccgacggc 360

gcagccggag ccagcagagc cggaaggcgc gccccgggca gagaaagccg agcagagctg 420

ggtggcgtct ccgggccgcc gctccgacgg gccagcgccc tccccatgtc cctgctccca 480

cgccgcgccc ctccggtcag catgaggctc ctggcggccg cgctgctcct gctgctgctg 540

gcgctgtaca ccgcgcgtgt ggacgggtcc aaatgcaagt gctcccggaa gggacccaag 600

atccgctaca gcgacgtgaa gaagctggaa atgaagccaa agtacccgca ctgcgaggag 660

aagatggtta tcatcaccac caagagcgtg tccaggtacc gaggtcagga gcactgcctg 720

caccccaagc tgcagagcac caagcgcttc atcaagtggt acaacgcctg gaacgagaag 780

cgcagggtct acgaagaata gggtgaaaaa cctcagaagg gaaaactcca aaccagttgg 840

gagacttgtg caaaggactt tgcagattaa aaaaaaaaaa aaaaaaaaaa aaaaaaaaaa 900

aaaaaaaaaa gcctttcttt ctcacaggca taagacacaa attatatatt gttatgaagc 960

actttttacc aacggtcagt ttttacattt tatagctgcg tgcgaaaggc ttccagatgg 1020

gagacccatc tctcttgtgc tccagacttc atcacaggct gctttttatc aaaaagggga 1080

aaactcatgc ctttcctttt taaaaaatgc ttttttgtat ttgtccatac gtcactatac 1140

atctgagctt tataagcgcc cgggaggaac aatgagcttg gtggacacat ttcattgcag 1200

tgttgctcca ttcctagctt gggaagcttc cgcttagagg tcctggcgcc tcggcacagc 1260

tgccacgggc tctcctgggc ttatggccgg tcacagcctc agtgtgactc cacagtggcc 1320

cctgtagccg ggcaagcagg agcaggtctc tctgcatctg ttctctgagg aactcaagtt 1380

tggttgccag aaaaatgtgc ttcattcccc cctggttaat ttttacacac cctaggaaac 1440

atttccaaga tcctgtgatg gcgagacaaa tgatccttaa agaaggtgtg gggtctttcc 1500

caacctgagg atttctgaaa ggttcacagg ttcaatattt aatgcttcag aagcatgtga 1560

ggttcccaac actgtcagca aaaaccttag gagaaaactt aaaaatatat gaatacatgc 1620

gcaatacaca gctacagaca cacattctgt tgacaaggga aaaccttcaa agcatgtttc 1680

tttccctcac cacaacagaa catgcagtac taaagcaata tatttgtgat tccccatgta 1740

attcttcaat gttaaacagt gcagtcctct ttcgaaagct aagatgacca tgcgcccttt 1800

cctctgtaca tataccctta agaacgcccc ctccacacac tgccccccag tatatgccgc 1860

attgtactgc tgtgttatat gctatgtaca tgtcagaaac cattagcatt gcatgcaggt 1920

ttcatattct ttctaagatg gaaagtaata aaatatattt gaaatgta 1968

<210> 23
<211> 16
<212> DNA
<213> Artificial

<220>
<223> Description of Artificial sequence: primer

<400> 23
gaagccaaag tacccg 16

<210> 24
<211> 17
<212> DNA
<213> Artificial

<220>
<223> Description of Artificial sequence: primer

<400> 24
ttcttcgtag accctgc 17

<210> 25
<211> 2462
<212> DNA
<213> Homo sapiens

<400> 25
gcggctgtct cacggaccac ggcggcgccc gcagctcctc accgcagcac ccccactgca 60

ggcccgagga gctttccgga gcttcccaca ctcctgggga gaagacttct tagccagctt 120

gatgtttaaa attcagctgg agcccttaaa acttcgagcg tggacgctga atgggtttgt 180

aaagtttcga aacaaggaga ccagtgctgg tccagtggct gtgatgggaa aagattatta 240

caagattctt gggatcccat cgggggccaa cgaggatgag atcaagaaag cctaccggaa 300

gatggccttg aagtaccacc cagacaagaa taaagaaccc aacgctgagg agaagtttaa 360

ggagattgca gaggcctatg atgtgctaag tgaccccaag aaacggggcc tgtatgacca 420

gtatggggag gaaggcctga agaccggcgg tggcacatca ggtggctcca gtggctcctt 480

tcactacacc tttcatgggg acccccatgc cacctttgcc tccttctttg gtggctccaa 540
ccccttcgat atcttctttg ccagcagccg ctccactcgg cccttcagtg gctttgaccc 600
agatgacatg gatgtggatg aagatgagga cccatttggc gctttcggcc gttttggctt 660
caatgggctg agtaggggtc caaggcgagc cccagaacca ctgtaccctc ggcgcaaggt 720
gcaggacccc ccagtggtgc acgagctgcg ggtgtccctg gaggagatct accatggctc 780
caccaagcgc atgaagatca caaggcgtcg cctcaaccct gatgggcgaa ctgtgcgcac 840
cgaggacaag atcctgcaca tagtcatcaa gcgtggctgg aaggaaggca ccaagatcac 900
cttccccaaa gaaggcgacg ccacacctga caacatccct gctgacatcg tctttgtgct 960
caaagacaag ccccatgcac acttccgccg agatggcacc aacgtgctct acagtgccct 1020
gatcagcctc aaggaggcgc tgtgtggctg cactgtgaac attcccacta tcgacggccg 1080
agtgatccct ttgccctgca atgatgtcat caagccaggc accgtgaaga gactccgtgg 1140
ggagggcctt cccttcccca aagtgccaac tcagcgagga gacctcattg ttgagttcaa 1200
agttcgcttc ccagacagat taacaccaca gacaagacag atccttaagc agcacctacc 1260
ctgttcctag gctctgcccc agccagtcca gagcctacca cagcaatacc cccaacactc 1320
actccactca atgtgcaccc agcttgatgt ccactggaca ctggcaactt tttctaaaat 1380
gcaaaaaaaa gccactggtt ttcaggaaaa tgttcctgtc cctgacccct tttagagctg 1440
ggctgcctgg ggggagtggg agggaggtgg ggagagctag cccaggccag gggtcaatgt 1500
tcatgtcact agctttcaat ccagtttcca ctgcagtggc tggagagtga cctgagtgct 1560
acttgaagat atgtagagat tccttatcca tgcctgtaca tagcatgtcc tcctcccctc 1620
agctttgcta ataccagtcc cctcccttcc ctttggcttc ctgctgttgg gggtggaaaa 1680
gactagaaag gacattgctt tctcagcccc acccccaggt ccacagtgtc caaggtaatg 1740
gcacacatat tcatgcacaa gaagcactca cctagagctg tctgtgcctc tctgggagaa 1800
aggagagagg ataagaaggg aaagttcaca acctgtgaac agggacttga gcacgagaca 1860
cttttcatct ggagcagggg ggtgagctcc tcagcagcct ccgtaacagc tgccctgcct 1920
acacctgcag agctggaggt tctgtcctcc ctgctgctct caggagtgtt caagggtgga 1980
gggcagggaa tggggcctga ggtattaagg ctaagaggtg ggggacaggg cccatctacc 2040
agccttcatc aggaagggaa agggctttgg ggtcaggtgg cagctattcc ccaccaagag 2100
attcagggtc acaggttttt ccccacacct ctgaactcag ggcctagcca cccccaaact 2160
tccaaatccc acttttgtga tatgtgaagc tactcatttc ttacccttgg aggctgtgtg 2220
gggaatttcc agccctttta tgcctgtgtg atcccacccc acccccatag ttgtataaag 2280
gtcatagtga agaagctggg ggaagactgc ttcagccaga tcctggggtg gggtctttag 2340
gttttctcac tttgacaacc cccgaatgtt tttatagtag tttttttgta tttttttgta 2400
atgacagtat tatgtaaaaa ataaagtatt ttaaaaatat ggaaaaaaaa aaaaaaaaaa 2460
aa 2462

<210>  26
<211>  16
<212>  DNA
<213>  Artificial

<220>
<223>  Description of Artificial sequence: primer

<400>  26
caacgtgctc tacagt                                                        16


<210>  27
<211>  16
<212>  DNA
<213>  Artificial

<220>
<223>   Description of Artificial sequence: primer

<400>  27
ggagtctctt cacggt                                                        16


<210>  28
<211>  18
<212>  DNA
<213>  Artificial

<220>
<223>  Description of Artificial sequence: primer

<400>  28
gaaggtgaag gtcggagt                                                      18


<210>  29
<211>  20
<212>  DNA
<213>  Artificial

<220>
<223>  Description of Artificial sequence: primer

<400>  29
gaagatggtg atgggatttc                                                    20


<210>  30
<211>  20
<212>  DNA
<213>  Artificial

<220>
<223>  Description of Artificial sequence: primer

<400>  30
cggctaccac atccaaggaa                                                    20


<210>  31
<211>  18
<212>  DNA
<213>  Artificial

<220>
<223>  Description of Artificial sequence: primer

<400>  31
gctggaatta ccgcggct                                                    18


<210>  32
<211>  13000
<212>  DNA
<213>  homo sapiens

<400>  32
tagagacact ctttgagaac cccaaattta aaatcattta gctggaagtt gttcacattt      60

cataaaatct gtatttcacc ccaggacctt ttaaacctcc ctaggcattt aaagtgggga     120

ttatacagcg tttatgaaac agtgtgcccc aaagaaagat gcgtacacag gattatgcag     180

tgtgactgtc attatgtgaa agtttctgtg cacgggcaca ggctggactg gttctgggaa     240

ggatggaaag ttccatgtgc ccctttttct cttcagtgat gctgtactgt cttcagaatg     300

gtatcatttg tttggttctt tgcttatttt tgcttgtttg tttgtttttt tgtttgtgaa     360

atgggagttg aaatggcctc aaacagacct aggcaccaaa tccaattttc cagtcaaatt     420

aattatgagt tcctgatgca gcattcaagg ctctccgcaa taccctcttg ctgcccacac     480

tccctgtgta tactctacct cccatcaaac cagccagtcc ctgactcatc acatgtgttc     540

tgacctcagg ctggacctca gctgccctgt ccctgcgggt caccttcact cacccctctc     600

tacctgagca gtcttcctca tccttcaagg tctatttcca ctacctgctt catgaaagca     660

ttcccaatcc ccaacctgga tgggactgcc ttacagcccc ccatctggaa tcctcgcatc     720

cgtaactcat ttgaagtatt ctctcagcct gctggcaagg gcctctctgg cctgcagaca     780

tcctgccgag cctctggccc gcagcccccg cagccctgca accccggcac agagcaggca     840

ctcagcaagg gctcaacttg gtgtttatag aggattttgc ttaccagaat aaaataaacc     900

ccccatttca ggccaaggat ggcaacttgg ataatcatgt ttttcatccc aatccaaatg     960

gatttccaca tgtgttagtg gctttttttc ctccacagga taaacaaatt tagtctcttt    1020

gtgatagctc aaatacatgt ttaaagaact gcccccaaat gtttcatctt caaagacaaa    1080

aactcaggaa aaacttgtac aatacttctg ttagtccagc tgaaacagta gctacaagaa    1140

cattaattct ccggcctctg ccttcttcct tggacactgg accgtcagcc acgacaccag    1200

gagcaaaccc gccctgcttt ccgtggttct gagcctcttg aattcgggcc tccgccaaca    1260

cacagcagga catctagcct tacacagggg cccgttctca gagtccccat accacacttt    1320

ccctcctgcc ctctctctct agcatccaca tctggtttcc cttcagcaca agtttcacaa    1380

tggacagatt ttgcacttcc aggatttatg gttcctgctg cccctccttg cttttgatat    1440

gattccatta tgtgcttaaa atctgggccg cactcttggc tctggggagc tgtttatgac    1500

aattctttta tggccttaaa tcgctcaccg ttaagaacct gatcatgttc agaggcaatg    1560

actagttgct aagaaagaaa attgcaacat gtctcagatg gttgcttgaa aagttttctt    1620

catagaagat tcatgaaggc tagtatttac ctggcaggta aatcctctga ggaacacctt    1680

ggaccctgtt ctcctccatc agctgccaga gactgattaa aagcaaatta gtcacaatct    1740

tctcacccca gacccaatca cttcctccct tctgctgtgc tagagaccac actgacagaa    1800

ttggtaaaaa taaaaacacc aatatcacaa acactccctc ttgacagcaa acgagactga 1860
gaacattttt tcttttccat ctgtgcatta cattttttca gaaggcaaag aatttcagga 1920
ataagaagac aaaaactttt atgaaagaca gggcatggtg gctcacacct gtagttccag 1980
aattttggga agcttagatg ggcagattgc ttgagctcag aaattcaaga cacgccaggg 2040
caacatagtg agatccccgt ctctacaaga aaatttaaaa actagccaag ggtagtggca 2100
tgtgcctgta gtcccagctg cttgggaggc tgaggtggga ggatcacttg cgcctgggag 2160
gcagaggttg caatgagctg agatcacacc actgcactcc aacctgagtg acagaacaag 2220
accctgtctc aaaaaaacaa aacaaaacaa aaaaaataca atgccctgac ctttgggcaa 2280
gaacttccct gaaccaaggt ttctcttgcc tcccatctcc ctctctgcca tccttctttc 2340
gtccagcagc cagacagatc ttctcataag gcaaaaccga atcacacaac cctgcttgaa 2400
attctccaat ggcttcttat aatccaggtg atgaagccca aactcctcag ctgcccagga 2460
ccctgcacaa tttggtgctg atctacatat tcagcctaat cacccttccc tcccctgccc 2520
agatccctgc cctacacaca cagggctccg ttagcatcct acagtttcct tgaccttccc 2580
catcccatag cagttgttta agctgttgtc tctgcctgaa ccatcctcct caatgcctgc 2640
ttccctatca gagcacgaat gcccactgtg ccctatgaga gcttagcagt gtgcctagcc 2700
tttctctccc cacattccgc ccatggcaga cccagaaggc tccatgtctg tgtctgagat 2760
cactgcagaa gactccttgg accaccacat taccaccctc tggtactgga tgtagggttg 2820
ccagttaaaa tacaggccac aagttaaatt tgaatttcag tataaacaat gaataatttt 2880
gagtataagt atgccgtaaa tattgcatgg gcattttgta ttttttgttt gttaaagttg 2940
acaatcctag ctggttgaga actctgggat gccctccagg gagaagaact gaggaccagg 3000
tggcagcagc atgcagacag gaaggtagtg ggtgacacct gggcaaaagg tagggagcgt 3060
ccccccaccc tcctcctggg aggcagcgta tggccgtgca gagcaggact ccagagtcag 3120
attgcttggg tttgaatctc agctctgcca cttggtagtt attttgggca acatacttaa 3180
ccttctcctc agtttcctca tctgtaaaat ggggataacc tactttgtgt ggttatgaaa 3240
attaaataaa ttaatatttg tatagcactt agaacagtcc ttgagacata gtaagcattc 3300
tataagcatt tcataaatat aaagcaaaag aaattagcca agccaaccca cagataaggg 3360
gagtgagtct acgagattta ataatcataa aacaagaatg aacatttatg gaacacttac 3420
tatgtgacaa gatactgtgc aacatgtttg ttctatatca gctcatttaa tccttatcac 3480
agcctgatta tttattatta ttattattat tatcttcttt ttacagatga ggaagcagaa 3540
gcacagagtg atagactagt actctagacc aggaagtctg actctgtaac acactagact 3600
gtgataaact gtgatccaca ttaattcatt catcctgcat tcgttcatcc tgcattcaat 3660
catttcttca ttgagcacct actgtgtgcc cggccctgtg ctcaagctgg ggaactagaa 3720
ttaataagaa agacacagag caacctcaga ggatcatgga cctgaaagaa gacctcatag 3780
gagcctttac aagtgtgagg agtggcactg gagacaacaa ggtctacgag ccacgcggac 3840

agcaggtgcc tctcctaccc tggggctagg acgggagtca ggcagatgtg tgcagtcaag 3900
gagggcttcc ctgtaaaatg acatcttcgc agtttcagga aggcaaacga aagcaaacag 3960
cctagccaag gagacaggac ttggcggtgc ctgcacagag gcctaggact ctgctgcaaa 4020
catggaacag ggctggtgag agaggcagga ggtggggcag agcagaatga ggacaagtga 4080
ggccggatgg aggggctgct tgttcccctg tgccaagcag tgggggacag ctctaaagat 4140
gggcctagac cctcaccatg ggactcaccc aagccaccaa caactctacc tgcctctctg 4200
cagtgccacc tgcacagcac aggtgctcat cgcccatcgc cagcgtctgc tcagtgaggc 4260
tgaaggctgc ccaagccctc gctgaatcaa ctgctcctga gttgctgtta taacaagaac 4320
attctgaaaa actggaaagc aaggagagaa gctttacaac aacaatgccc aacatttcgc 4380
cactcaaaga gaatgacgtt tgtaactctg tcttgccatc ctttgtacag aggttactgt 4440
tttgcatact tataatcaca gtgtttacac aaccttgtgt tttttctact tactgcataa 4500
gcatttactc cacaaattta gagctttcat tgttttctta atcccccatt gttgggcatt 4560
tagtttaaat ctttattttt aacaaggact agctttgcat gtggtatttc tccattatga 4620
gaattttaaa cagatttctg aagtggttaa taagatgaaa tggtttactt caaacagtct 4680
ggataccata ctataaatat aacttcccca aattttgtac caattgatag tcccatgagt 4740
gatgcttaag tgatgcttcc tcactttacc attatcagct ttggatgttg ctatttaaaa 4800
taaacatttc ctcattgagt gggaccaaaa ccaaaatgcc gtgatcctgc agcactgctg 4860
tgcttttcct tcattgccaa agaggatgca tgttctttga tgaattggtt aattgccttt 4920
ctttttctcc tgaattgtcc tttgatgtcc tttgctcatt tatatgttag ggccctcttc 4980
ggaaatatct ttgcatgtgt tcttaatata ataaagatgc ttgcctgtaa gggagtgttg 5040
ttggatgaat agatggtgga ataaatgcta cttgttggtt tgtcatattt gctgcaagca 5100
ttttctccca tgtttagggg aatcttgcag tgccccctgt gacccatctg agtaacagag 5160
cccccctaca tacactctgc ctcggggctg ctttaagcct aattcaatac tgcctggctg 5220
tcccccacat cattttctct gggcttaagt tactcaggat ctctaaatat ttgcagtgtg 5280
aagaacaaaa ccagaggttg ttcttgggaa ccttggtcct tcaacttcct tcaccccaga 5340
gcatggccag ggaaggggtc agcatgcagg gctgtagctg aaagaaagct gcacagagga 5400
ggcagctggc tgcccagact ccacaggctg caggtgaaca gacagaggga ggagctggag 5460
agggagaggc acccgccagg cttggattca gagccagcag ccacacaaca caatggaagc 5520
aggtattttg gggtgaccca ccatgtcatg agtggtgatg gggccagaca ggttactgga 5580
gcccaggatc ttgctctggc ctaagtagct aggtacagga cacagagaca ggaggctggc 5640
taacattaat gcacagggag atcttgtatg cagtcaaccc cattgctggg cctcagtttc 5700
ctcatatgta caatgaaggc cagttgtggc ctcagcctct accagtgctg aattctagga 5760
ttcctgtcct gccagggaag taaaaaggac tcctcttctg aaaagaaggt cagtcacgaa 5820
aagaactaaa agaatggggg aaggaattgc cctggatccc agctcccagg gcagaaaacc 5880
agccttgacc agtcaagacc cctttctaag cagaccaagg aggtagtgcc cagacacctg 5940

tgggatttcc tggaaagtca cactcctgtt atcaccacct cctcttccca gagacttcat 6000
ctctaaaaca aatgccctga ggatgaactg gcttccctgg gaatatggac tccccatggc 6060
cacccctaca cacacgcaaa cacacacaca cacacacgca cacacacttt ctcacatgca 6120
accttgtagc ctgtgcagcc tggattcagc agtactctca catgctgagc tgttgcctga 6180
aacatgtttt tatttactcc acagataggg agtgaacccc tgctacatgc caatcactgt 6240
tgtagatact gaggatgagc aagacagaca cccacagaca cagacagatc atccacaatg 6300
cacccccaaa taaatatgaa aatgtacaat ttgaggttgc tgcacataca atgaataaac 6360
taaaatcggt ggcaacacag agagggactg ttgttggggg tatgctcgtg ggattagaac 6420
tggatggtga atattggagc ccaggctcac tgaggagaag agggttgcct gtcagcccct 6480
ggggaggagc actgcaggtg gaggggccag tgagcacaaa gaccctgagg tgggaacagg 6540
cttggatgtt tgaaggtaag acaaggcaga gtatctggag cacagcctgt ggggagcatg 6600
gcaggtgctg gtcatgattg ggtgcgcact gctggggact tggcttccac tctgcatgag 6660
atcgggagcc agtaaagggt tcagagcagg gacttccccc aggagatcac tgtgtgagca 6720
gcagggacca tgaagggcca ggagtggcct ccctcctgtg gtcactggtt aattcctttc 6780
catccttcat atctaactca atggcccttt cctccaggaa tttgtcccca cctcgtcacc 6840
ctgtcagatc ccctgatgag tctcctagca ccaaccaccc tccaggacac tgacctctgc 6900
aggagtttca cactgatctc tgtggtttat agctaatgtc tgccttcccc actagactct 6960
gagctgcttg ggacaggcca cacagctgcc ttggctcacc tcaaaccccc agcacctcgt 7020
acaacgcctg ggacacagtg ggtgctcaat aaatgtaaag agaagaaata agcaaatccc 7080
tggggtctca gcaaaatttc cactccctca aggagtgatg tcctgtttcc acttcctccc 7140
tcctctgtgc ccccacactt cacgtctacc tcttctgtag aatccagtat gaccatgaat 7200
gtgttgacat ctgcacacat gcatacacac acacaggcaa ggcatgtgtg ctgtccttca 7260
ctgtacccca gggacctcct tgaagcagac agtgccactg tacaaagaac ggcctcacac 7320
atgcccacct gctcagaggc cctcggcttg ggctcactag acagtctgtg ccctgaaggt 7380
gtgtcagggg ccaagccccc tccctcaccc agttaggcag catcccccac agacctgctg 7440
gcagcagggg cctcatgctc tcctgtacca tggctccaga agaactcaca caaacaaggg 7500
ctcagctctg cttgcacagt ggctgccttc tgcaaaccag agatctgcat acacagactg 7560
aggagtgtaa ctgaggacaa aacactggga cgtctgacct ctggtccagc ccaagaagct 7620
ggggaacgtt ggtccatatt tctagagcca actacttttt cctgtgaaaa ctcagtgccc 7680
acgatcgctg gtgttgttag atcccagtta atcaatcaac atattcttga aacaaacatg 7740
ttagaaataa aatcgttaat aaaaaataat actttacatg ctctaggaaa gcttggtatt 7800
taagaaattc ctttgcagca taaaatatat ttttttggaa agcaattggc aatttctatg 7860
aaaagtctta aaatgggcat gtgccatggc cccatgattc tatttctgag ggctgatcca 7920
atgtattaaa acctacccca aaatggagct acaagaatat ttgtcaaaca gcattggttg 7980

taagagggta aggttgaaaa caatccaaat gtccagcaag gagtagtcaa attaattctg 8040
gtacatctgt atgacggaat actctgcagc taaaatcatg ataaccgcaa tggtcagcac 8100
atatcaaaca cttactctgt gttggggacc acattaaaag cattacatgc actatctcat 8160
ttaattctgc ctatgctgta catattatta tcatctccat ctcacagatg aggaaaccaa 8220
taccaaatag agttaaggta tttgcccaaa gtcacacagc tagtccctag gatgcatact 8280
caggcaattt gactccagac accagcctct tagccattat actacccggc ctcagatgaa 8340
accattaagg gagctattgt tgtagcaagg ctagccacat ggagcgatgt tcacaatatg 8400
ctaataggta tagcaagcag gttattaaag actacatgca gcctgatccc atgtgtacat 8460
gtgcacacaa agcaaaaaga ggatgctttg ggaagatttc acccaagatt ttacttgcgt 8520
gaaaatgtta gttttcttct gcttgggttt tctgattttt ctgggcaaac gtgttgtttc 8580
tatattttaa aaaattattt ttagagttaa aaactaacat aaatgccaaa agtcactccc 8640
tcatactcat ctcatgcata aatgcaaatg tccagctatt ggctttgctt taggtatagg 8700
cacaacagca cgcataggcg ctgcttgctt ttcatggctc agcttcttct tgaagcagca 8760
agggctctcc agttgggaag agacgacaca gattcttccc attgcaccct tcagcagact 8820
gtccccatca tccagaagcc ctctgggcat ttttgcctcc agggaactgc caggctgatg 8880
gcatagagct gggtgagctc tctcctgaca gccaagccca gggactccaa gagcttgaga 8940
tgatccacct cctgtctctg ggtctctcaa ggcagcaaga agtgaagggc agacaggcct 9000
caaagcaaca gagtggtcca gggacaggag tcccagttct gcagccgtat atactgagga 9060
ccctcctggc ctagtcacct gcttcctagc tctgtgacct tggacaagtt atttcttctc 9120
tcagagcatg tttccacacc tgtcaaataa agaaattaaa agtaccaact tggaagaaaa 9180
gtaccaattg gtaagaatca aagtaataga taaattagac ataaaacgat ggtgccaata 9240
agtaacattt cctgggtact gacaacatgt atgatttcta tagttctcac agccacccta 9300
tcagaaacac tgtgatgatc tgcactttac agatgatgaa acaaaagctg agagcggtaa 9360
agtcacttgt gctggaaagt agtgaggagg gaatttctgc cactatggct gggctcacac 9420
cactatacct cgcattctgg cacaatgtct agaacatggg aaggccttca tccagatggt 9480
taccactgtc atcgctctcc actagggctc tctacctggc tttggagagt agtgtgagag 9540
tgaggactcc tcctggttct ccccaccacc cagacactca cacagccacc aggccactga 9600
cccactttaa ggagctttcc tttgggagtc accaaaactc ctccccaata cccgtcccca 9660
cccccgcata cacacacata ccctctttga gtcagttcca gtgatcacag tcaatgactt 9720
tgctgctgga cagtctatga caagcccctg tccagaactg gccttgtgtt ctggcccagc 9780
aggggccctt gggggtcagg cctgcaggca ctcacacttg gcacctgctc caaaaccctt 9840
tcaggtcttt gaggatctga gccctgggcc tgggtctccc gccggctcgg aaaagctggc 9900
ctgccgggcc agagagagaa ccacacgatt cagaaaagca gtgcccttca gcagcctctc 9960
caccgtctgg gctccccaaa ggcagagcgg gacgctggaa atgtgtgcgc gctgtggtat 10020
gggtgtgcaa gtgtgcgaag gcggcgtgtt gtgtgagcga gagggtagcg gatgtgtgtg 10080

tgcgtgtgcg cgcgtggctc cgggtgtgcg ccgctgcgat agcgggtcct ttcccggggc 10140
gggcgacggg cgggctggga aggtctcctc ccctcaccac attgagaaat ctcagtgagt 10200
caccgagtgg ttctgcatat taatgagctc gctcgctgcg agggcaggag cggatttaaa 10260
agaggccagg gcgggcggag ggaggctgtg gagagagcgc ggagacaagc gcagagcgca 10320
gcgcacggcc acagacagcc ctgggcatcc accgacggcg cagccggagc cagcagagcc 10380
ggaaggcgcg ccccgggcag agaaagccga gcagagctgg gtggcgtctc cgggccgccg 10440
ctccgacggg ccagcgccct ccccatgtcc ctgctcccac gccgcgcccc tccggtcagc 10500
atgaggctcc tggcggccgc gctgctcctg ctgctgctgg cgctgtacac cgcgcgtgtg 10560
gacggtgagt gcggggagct ccccttgtcg ccgtctcgtc ttgtcctgtc ctggggaacc 10620
caagcgccaa tttctaggca tcctgggatc ccgcgggggg tggtctgggt gggcaccaag 10680
gtggtcgagg ggcaggagac cgcctagcaa ctaagccgtt gccccgcgct cgctcgcagg 10740
gtccaaatgc aagtgctccc ggaagggacc caagatccgc tacagcgacg tgaagaagct 10800
ggaaatgaag ccaaagtacc cgcactgcga ggagaagatg gttatgtgag ttctcgctcc 10860
tcaccttgag ggggtgcgca ctgctgtcaa cccaatgcca gggcacagcc tgggcctggg 10920
tcgggctaac aaacatttct ctccgacttt gtcaaggccc tggaaaagcc tttgtaattg 10980
cccagagagt ccttaggacg ctggcgagac actctggaag acagtcggga aggggggtca 11040
ctgtcccgct ccggaacagc gcctccaagt tcacccttgc taggattcaa atgagcagtc 11100
tccctatttg taccatgggc cacccactct gctagggcca tcaagggtta aagggttccc 11160
ttgtgccaac tcagaaaacc tagactgaaa tcacttaaaa cacactcaca cccaaactgt 11220
gaggcgtgct gccttcctgt cactggatag aggactaatg caaacactat cttaaatcct 11280
gagtctcctt cagagccttg ttccagtcag agacaagtgg accgcaagtt tttggctcct 11340
agagatttta ttttgagaac gctccacgag cttccctcac cgactgtact ccttgtgggt 11400
ccccaatacg gagcagtact gcagaaagtg ctctgctggg gttaatgtgc cacatggctg 11460
gagccctgct tggtggggat gtggggaatg ttggaaaaac tggggcagag tgagagaaca 11520
cttagtttct tgctcctccg ccttcccaat taaccacttg aggccagcac cgctgcctcc 11580
tcatcaatat gctcttggcc aggaagggag cccttggcaa agcggctacc aaatatttgc 11640
cacagaccat tggcccctct tcccctcccc cagaaacagg acgcacttta ttctcagtct 11700
gggcaagtgc cctccaccag ctggccccca ccggcgcaaa ggccttcaca gtgtctgcga 11760
aagcagtgca cacccctacc cagcaccccc catccccgtc taggggctac actgtcaggc 11820
tgaaattagg ggaattcaga aatccactgg aactgagctg accccactgc cttccttcac 11880
agacacctaa attaacttgc ttgtccctac cccttgcctt gcagaccaaa ttcaaagaaa 11940
atacccaaaa atccttttgg gtgggggcca agtgtcaaat gagctgggaa aggagctcca 12000
agctcctcag ggcttccagt ttgggggcta agaatgacag gggaattcca agttaaatct 12060
cactcgtcta agagctgaaa accactctat taaccaaaca agtttctgag accttgcagg 12120

ggaagaaagc agggtaaagg atgcagtgat acctaaaaag gacagagccc tcagtaaata 12180

ggcttgctct tttcctggct aaacttttcc acatatagag aaaaactata gctaggcaag 12240

tgtgagaaag gctttacgga gccttgtaga aacgtgcagg ggcctcagga gatcagcaca 12300

caatccaatc accctctact aaacctctaa cccaccccct agggcctccc actccatccc 12360

tgagggctgg gggctcctct ccccctccac aatcctaaaa ctctccaaat cagatgaggg 12420

gccagcactt ttgcaaagca ggttttcagc cacaaaggct gaacaaaagc aaattttcca 12480

gcttaccctg agtcacccag aaatctctgc tgaggctggg ggtgggggca ggagtagggg 12540

cggggggaga ggtccgcttt gcaagtggga gtccttgcac ccagaaggat ccaggagagt 12600

ccgaattcta gaaagagcaa tttgtgtcat aggtccatgg agctctcacc aaaaccctta 12660

cccccaccgc cagaactgtg gaggagaggg tcacaggact cagaatttct gatagttgct 12720

ggagtcacaa atccctatgt ctgcactcca caaccgattt tcataatccc agtattatga 12780

cgtggcattt gagcactgta gtgagagggc tgagtgaccc ttcctgctct cccttcctgc 12840

tctccctgac cttcaggatt tgggatgcac agcctgcaca ctgacctaac tgagaaaggg 12900

tttttatcag gcattaccac gtcctttcct tcagaatgct aaagcagcct tgtaggcaga 12960

aagagaaaag catttgctaa attcctgcag ggtgactggg 13000

<210> 33
<211> 4818
<212> DNA
<213> Artificial

<220>
<223> Description of Artificial sequence: plasmid

<400> 33
ggtaccgagc tcttacgcgt gctagcccgg gctcgagatc tgcgatctaa gtaagcttgg 60

cattccggta ctgttggtaa agccaccatg gaagacgcca aaaacataaa gaaaggcccg 120

gcgccattct atccgctgga agatggaacc gctggagagc aactgcataa ggctatgaag 180

agatacgccc tggttcctgg aacaattgct tttacagatg cacatatcga ggtggacatc 240

acttacgctg agtacttcga aatgtccgtt cggttggcag aagctatgaa acgatatggg 300

ctgaatacaa atcacagaat cgtcgtatgc agtgaaaact ctcttcaatt ctttatgccg 360

gtgttgggcg cgttatttat cggagttgca gttgcgcccg cgaacgacat ttataatgaa 420

cgtgaattgc tcaacagtat gggcatttcg cagcctaccg tggtgttcgt ttccaaaaag 480

gggttgcaaa aaattttgaa cgtgcaaaaa aagctcccaa tcatccaaaa aattattatc 540

atggattcta aaacggatta ccagggattt cagtcgatgt acacgttcgt cacatctcat 600

ctacctcccg gttttaatga atacgatttt gtgccagagt ccttcgatag ggacaagaca 660

attgcactga tcatgaactc ctctggatct actggtctgc ctaaaggtgt cgctctgcct 720

catagaactg cctgcgtgag attctcgcat gccagagatc ctatttttgg caatcaaatc 780

attccggata ctgcgatttt aagtgttgtt ccattccatc acggttttgg aatgtttact 840

acactcggat atttgatatg tggatttcga gtcgtcttaa tgtatagatt tgaagaagag 900

ctgtttctga ggagccttca ggattacaag attcaaagtg cgctgctggt gccaacccta 960
ttctccttct tcgccaaaag cactctgatt gacaaatacg atttatctaa tttacacgaa 1020
attgcttctg gtggcgctcc cctctctaag gaagtcgggg aagcggttgc caagaggttc 1080
catctgccag gtatcaggca aggatatggg ctcactgaga ctacatcagc tattctgatt 1140
acacccgagg gggatgataa accgggcgcg gtcggtaaag ttgttccatt ttttgaagcg 1200
aaggttgtgg atctggatac cgggaaaacg ctgggcgtta atcaaagagg cgaactgtgt 1260
gtgagaggtc ctatgattat gtccggttat gtaaacaatc cggaagcgac caacgccttg 1320
attgacaagg atggatggct acattctgga gacatagctt actgggacga agacgaacac 1380
ttcttcatcg ttgaccgcct gaagtctctg attaagtaca aaggctatca ggtggctccc 1440
gctgaattgg aatccatctt gctccaacac cccaacatct tcgacgcagg tgtcgcaggt 1500
cttcccgacg atgacgccgg tgaacttccc gccgccgttg ttgttttgga gcacggaaag 1560
acgatgacgg aaaaagagat cgtggattac gtcgccagtc aagtaacaac cgcgaaaaag 1620
ttgcgcggag gagttgtgtt tgtggacgaa gtaccgaaag gtcttaccgg aaaactcgac 1680
gcaagaaaaa tcagagagat cctcataaag gccaagaagg gcggaaagat cgccgtgtaa 1740
ttctagagtc ggggcggccg gccgcttcga gcagacatga taagatacat tgatgagttt 1800
ggacaaacca caactagaat gcagtgaaaa aaatgcttta tttgtgaaat ttgtgatgct 1860
attgctttat ttgtaaccat tataagctgc aataaacaag ttaacaacaa caattgcatt 1920
cattttatgt ttcaggttca gggggaggtg tgggaggttt tttaaagcaa gtaaaacctc 1980
tacaaatgtg gtaaaatcga taaggatccg tcgaccgatg cccttgagag ccttcaaccc 2040
agtcagctcc ttccggtggg cgcggggcat gactatcgtc gccgcactta tgactgtctt 2100
ctttatcatg caactcgtag gacaggtgcc ggcagcgctc ttccgcttcc tcgctcactg 2160
actcgctgcg ctcggtcgtt cggctgcggc gagcggtatc agctcactca aaggcggtaa 2220
tacggttatc cacagaatca ggggataacg caggaaagaa catgtgagca aaaggccagc 2280
aaaaggccag gaaccgtaaa aaggccgcgt tgctggcgtt tttccatagg ctccgccccc 2340
ctgacgagca tcacaaaaat cgacgctcaa gtcagaggtg gcgaaacccg acaggactat 2400
aaagatacca ggcgtttccc cctggaagct ccctcgtgcg ctctcctgtt ccgaccctgc 2460
cgcttaccgg atacctgtcc gcctttctcc cttcgggaag cgtggcgctt tctcatagct 2520
cacgctgtag gtatctcagt tcggtgtagg tcgttcgctc caagctgggc tgtgtgcacg 2580
aaccccccgt tcagcccgac cgctgcgcct tatccggtaa ctatcgtctt gagtccaacc 2640
cggtaagaca cgacttatcg ccactggcag cagccactgg taacaggatt agcagagcga 2700
ggtatgtagg cggtgctaca gagttcttga agtggtggcc taactacggc tacactagaa 2760
gaacagtatt tggtatctgc gctctgctga agccagttac cttcggaaaa agagttggta 2820
gctcttgatc cggcaaacaa accaccgctg gtagcggtgg tttttttgtt tgcaagcagc 2880
agattacgcg cagaaaaaaa ggatctcaag aagatccttt gatcttttct acggggtctg 2940

acgctcagtg gaacgaaaac tcacgttaag ggattttggt catgagatta tcaaaaagga 3000
tcttcaccta gatcctttta aattaaaaat gaagttttaa atcaatctaa agtatatatg 3060
agtaaacttg gtctgacagt taccaatgct taatcagtga ggcacctatc tcagcgatct 3120
gtctatttcg ttcatccata gttgcctgac tccccgtcgt gtagataact acgatacggg 3180
agggcttacc atctggcccc agtgctgcaa tgataccgcg agacccacgc tcaccggctc 3240
cagatttatc agcaataaac cagccagccg gaagggccga gcgcagaagt ggtcctgcaa 3300
ctttatccgc ctccatccag tctattaatt gttgccggga agctagagta agtagttcgc 3360
cagttaatag tttgcgcaac gttgttgcca ttgctacagg catcgtggtg tcacgctcgt 3420
cgtttggtat ggcttcattc agctccggtt cccaacgatc aaggcgagtt acatgatccc 3480
ccatgttgtg caaaaaagcg gttagctcct tcggtcctcc gatcgttgtc agaagtaagt 3540
tggccgcagt gttatcactc atggttatgg cagcactgca taattctctt actgtcatgc 3600
catccgtaag atgcttttct gtgactggtg agtactcaac caagtcattc tgagaatagt 3660
gtatgcggcg accgagttgc tcttgcccgg cgtcaatacg ggataatacc gcgccacata 3720
gcagaacttt aaaagtgctc atcattggaa aacgttcttc ggggcgaaaa ctctcaagga 3780
tcttaccgct gttgagatcc agttcgatgt aacccactcg tgcacccaac tgatcttcag 3840
catcttttac tttcaccagc gtttctgggt gagcaaaaac aggaaggcaa aatgccgcaa 3900
aaaagggaat aagggcgaca cggaaatgtt gaatactcat actcttcctt tttcaatatt 3960
attgaagcat ttatcagggt tattgtctca tgagcggata catatttgaa tgtatttaga 4020
aaaataaaca aataggggtt ccgcgcacat ttccccgaaa agtgccacct gacgcgccct 4080
gtagcggcgc attaagcgcg gcgggtgtgg tggttacgcg cagcgtgacc gctacacttg 4140
ccagcgccct agcgcccgct cctttcgctt tcttcccttc ctttctcgcc acgttcgccg 4200
gctttccccg tcaagctcta aatcgggggc tccctttagg gttccgattt agtgctttac 4260
ggcacctcga ccccaaaaaa cttgattagg gtgatggttc acgtagtggg ccatcgccct 4320
gatagacggt ttttcgccct ttgacgttgg agtccacgtt ctttaatagt ggactcttgt 4380
tccaaactgg aacaacactc aaccctatct cggtctattc ttttgattta taagggattt 4440
tgccgatttc ggcctattgg ttaaaaaatg agctgattta acaaaaattt aacgcgaatt 4500
ttaacaaaat attaacgctt acaatttgcc attcgccatt caggctgcgc aactgttggg 4560
aagggcgatc ggtgcgggcc tcttcgctat tacgccagcc caagctacca tgataagtaa 4620
gtaatattaa ggtacgggag gtacttggag cggccgcaat aaaatatctt tattttcatt 4680
acatctgtgt gttggttttt tgtgtgaatc gatagtacta acatacgctc tccatcaaaa 4740
caaaacgaaa caaaacaaac tagcaaaata ggctgtcccc agtgcaagtg caggtgccag 4800
aacatttctc tatcgata 4818

<210> 34
<211> 751
<212> DNA
<213> Human papillomavirus type 16

<400>  34
agtattgtat gtatgttgaa ttagtgttgt ttgttgtgta tatgtttgta tgtgcttgta      60

tgtgcttgta aatattaagt tgtatgtgtg tttgtatgta tggtataata aacacgtgtg     120

tatgtgtttt taaatgcttg tgtaactatt gtgtcatgca acataaataa acttattgtt     180

tcaacaccta ctaattgtgt tgtggttatt cattgtatat aaactatatt tgctacatcc     240

tgtttttgtt ttatatatac tatattttgt agcgccaggc ccattttgta gcttcaaccg     300

aattcggttg catgcttttt ggcacaaaat gtgttttttt aaatagttct atgtcagcaa     360

ctatggttta aacttgtacg tttcctgctt gccatgcgtg ccaaatccct gttttcctga     420

cctgcactgc ttgccaacca ttccattgtt ttttacactg cactatgtgc aactactgaa     480

tcactatgta cattgtgtca tataaaataa atcactatgc gccaacgcct tacataccgc     540

tgttaggcac atatttttgg cttgttttaa ctaacctaat tgcatatttg gcataaggtt     600

taaacttcta aggccaacta aatgtcaccc tagttcatac atgaactgtg taaaggttag     660

tcatacattg ttcatttgta aaactgcaca tgggtgtgtg caaaccgatt ttgggttaca     720

catttacaag caacttatat aataatacta a                                    751

<210>  35
<211>  412
<212>  DNA
<213>  Artificial

<220>
<223>  Description artificial sequence: promoter

<400>  35
gcgcagcacc atggcctgaa ataacctctg aaagaggaac ttggttaggt accttctgag      60

gcggaaagaa ccagctgtgg aatgtgtgtc agttagggtg tggaaagtcc ccaggctccc     120

cagcaggcag aagtatgcaa agcatgcatc tcaattagtc agcaaccagg tgtggaaagt     180

ccccaggctc cccagcaggc agaagtatgc aaagcatgca tctcaattag tcagcaacca     240

tagtcccgcc cctaactccg cccatcccgc ccctaactcc gcccagttcc gcccattctc     300

cgccccatgg ctgactaatt ttttttattt atgcagaggc cgaggccgcc tcggcctctg     360

agctattcca gaagtagtga ggaggctttt ttggaggcct aggcttttgc aa             412

<210>  36
<211>  3705
<212>  DNA
<213>  Artificial

<220>
<223>  Description of Artificial sequence: plasmid

<400>  36
agatctgcgc agcaccatgg cctgaaataa cctctgaaag aggaacttgg ttaggtacct      60

tctgaggcgg aaagaaccag ctgtggaatg tgtgtcagtt agggtgtgga aagtccccag     120

gctccccagc aggcagaagt atgcaaagca tgcatctcaa ttagtcagca accaggtgtg     180

gaaagtcccc aggctcccca gcaggcagaa gtatgcaaag catgcatctc aattagtcag     240

caaccatagt cccgccccta actccgccca tcccgcccct aactccgccc agttccgccc 300
attctccgcc ccatggctga ctaatttttt ttatttatgc agaggccgag gccgcctcgg 360
cctctgagct attccagaag tagtgaggag gcttttttgg aggcctaggc ttttgcaaaa 420
agcttgattc ttctgacaca acagtctcga acttaagctg cagaagttgg tcgtgaggca 480
ctgggcaggt aagtatcaag gttacaagac aggtttaagg agaccaatag aaactgggct 540
tgtcgagaca gagaagactc ttgcgtttct gataggcacc tattggtctt actgacatcc 600
actttgcctt tctctccaca ggtgtccact cccagttcaa ttacagctct taaggctaga 660
gtacttaata cgactcacta taggctagcc accatgactt cgaaagttta tgatccagaa 720
caaaggaaac ggatgataac tggtccgcag tggtgggcca gatgtaaaca aatgaatgtt 780
cttgattcat ttattaatta ttatgattca gaaaaacatg cagaaaatgc tgttattttt 840
ttacatggta acgcggcctc ttcttattta tggcgacatg ttgtgccaca tattgagcca 900
gtagcgcggt gtattatacc agaccttatt ggtatgggca aatcaggcaa atctggtaat 960
ggttcttata ggttacttga tcattacaaa tatcttactg catggtttga acttcttaat 1020
ttaccaaaga agatcatttt tgtcggccat gattggggtg cttgtttggc atttcattat 1080
agctatgagc atcaagataa gatcaaagca atagttcacg ctgaaagtgt agtagatgtg 1140
attgaatcat gggatgaatg gcctgatatt gaagaagata ttgcgttgat caaatctgaa 1200
gaaggagaaa aaatggtttt ggagaataac ttcttcgtgg aaaccatgtt gccatcaaaa 1260
atcatgagaa agttagaacc agaagaattt gcagcatatc ttgaaccatt caaagagaaa 1320
ggtgaagttc gtcgtccaac attatcatgg cctcgtgaaa tcccgttagt aaaaggtggt 1380
aaacctgacg ttgtacaaat tgttaggaat tataatgctt atctacgtgc aagtgatgat 1440
ttaccaaaaa tgtttattga atcggaccca ggattctttt ccaatgctat tgttgaaggt 1500
gccaagaagt ttcctaatac tgaatttgtc aaagtaaaag gtcttcattt ttcgcaagaa 1560
gatgcacctg atgaaatggg aaaatatatc aaatcgttcg ttgagcgagt tctcaaaaat 1620
gaacaataat tctagagcgg ccgcttcgag cagacatgat aagatacatt gatgagtttg 1680
gacaaaccac aactagaatg cagtgaaaaa aatgctttat ttgtgaaatt tgtgatgcta 1740
ttgctttatt tgtaaccatt ataagctgca ataaacaagt taacaacaac aattgcattc 1800
attttatgtt tcaggttcag ggggaggtgt gggaggtttt ttaaagcaag taaaacctct 1860
acaaatgtgg taaaatcgat aaggatccag gtggcacttt tcggggaaat gtgcgcggaa 1920
cccctatttg tttatttttc taaatacatt caaatatgta tccgctcatg agacaataac 1980
cctgataaat gcttcaataa tattgaaaaa ggaagagtat gagtattcaa catttccgtg 2040
tcgcccttat tccctttttt gcggcatttt gccttcctgt ttttgctcac ccagaaacgc 2100
tggtgaaagt aaaagatgct gaagatcagt tgggtgcacg agtgggttac atcgaactgg 2160
atctcaacag cggtaagatc cttgagagtt ttcgccccga agaacgtttt ccaatgatga 2220
gcacttttaa agttctgcta tgtggcgcgg tattatcccg tattgacgcc gggcaagagc 2280

aactcggtcg ccgcatacac tattctcaga atgacttggt tgagtactca ccagtcacag 2340

aaaagcatct tacggatggc atgacagtaa gagaattatg cagtgctgcc ataaccatga 2400

gtgataacac tgcggccaac ttacttctga caacgatcgg aggaccgaag gagctaaccg 2460

cttttttgca caacatgggg gatcatgtaa ctcgccttga tcgttgggaa ccggagctga 2520

atgaagccat accaaacgac gagcgtgaca ccacgatgcc tgtagcaatg gcaacaacgt 2580

tgcgcaaact attaactggc gaactactta ctctagcttc ccggcaacaa ttaatagact 2640

ggatggaggc ggataaagtt gcaggaccac ttctgcgctc ggcccttccg gctggctggt 2700

ttattgctga taaatctgga gccggtgagc gtgggtctcg cggtatcatt gcagcactgg 2760

ggccagatgg taagccctcc cgtatcgtag ttatctacac gacggggagt caggcaacta 2820

tggatgaacg aaatagacag atcgctgaga taggtgcctc actgattaag cattggtaac 2880

tgtcagacca agtttactca tatatacttt agattgattt aaaacttcat ttttaattta 2940

aaaggatcta ggtgaagatc ctttttgata atctcatgac caaaatccct taacgtgagt 3000

tttcgttcca ctgagcgtca gaccccgtag aaaagatcaa aggatcttct tgagatcctt 3060

tttttctgcg cgtaatctgc tgcttgcaaa caaaaaaacc accgctacca gcggtggttt 3120

gtttgccgga tcaagagcta ccaactcttt ttccgaaggt aactggcttc agcagagcgc 3180

agataccaaa tactgttctt ctagtgtagc cgtagttagg ccaccacttc aagaactctg 3240

tagcaccgcc tacatacctc gctctgctaa tcctgttacc agtggctgct gccagtggcg 3300

ataagtcgtg tcttaccggg ttggactcaa gacgatagtt accggataag gcgcagcggt 3360

cgggctgaac ggggggttcg tgcacacagc ccagcttgga gcgaacgacc tacaccgaac 3420

tgagatacct acagcgtgag ctatgagaaa gcgccacgct tcccgaaggg agaaaggcgg 3480

acaggtatcc ggtaagcggc agggtcggaa caggagagcg cacgagggag cttccagggg 3540

gaaacgcctg gtatctttat agtcctgtcg ggtttcgcca cctctgactt gagcgtcgat 3600

ttttgtgatg ctcgtcaggg gggcggagcc tatggaaaaa cgccagcaac gcggcctttt 3660

tacggttcct ggccttttgc tggccttttg ctcacatggc tcgac 3705

<210> 37
<211> 13000
<212> DNA
<213> homo sapiens

<400> 37
   aacatgcagt catgtttttt tattgttgtt gttaattcat aaactttatt tttagagcag 60
   ttttaggttc acaaaaaagt tgtgctaaaa atatagagag tttttgtata cccctgtgca 120
   cacacataca tagcatcccc ccaaaagtgg tgtcttttta cgactgatta ccaacattga 180
   cacatcatta tcacccaaag tccatagttt atgttagggt tccctcttgg tgttgtacat 240
   tcaatgggtt tttacaaatg taaaatgaca ggtatccacc actgaaatat ggtatagaat 300
   agtttatcag ccctaaaaat cctctatgtt ctgcctattc atctctcctt cccgccaacc 360
   actggcagcc actgatcacc ttaccgtctc catagatttg ccttttccag aatgtcatat 420
   atagtcatgc atcacttaac aatgaaaata tgatctgaga tgtgtgtcat taggtgaatt 480
   tgtcattgta tggtcataac agagtgaact tacacaagcc aaaatgatat agcctactat 540
   acatctaagt tatatggtat agtctatttt tcctaggcta cagacctatc tgacatgtta 600
   ctgtactgaa tactgtaggc aattataaca tagtggtaaa tatttgtgta tttaaacata 660
   gaaaaagtac agtaaaatta tggtattata atcttatgaa accactattg tatatgggtg 720
   ccattgttga ctgaaatgtc attacgtggc acatgactgc agcaggaatc ataaagtatg 780
   tagccttttc agattggctt ctttcactta gcaacatgca cataagtgtc ctccatgtca 840
   gttcatggct tgatagcata tttcttttta gcacagaata acaatccatt gtccacatgt 900
   accatggttt atttatccac tcatccacat gaagacatct tagttgattc taagttaggg 960
   aagttatgaa taaagctgtt ataaatattc atgagcagat ttatgtggac aacagtgttc 1020
   aactcatttg ggtaagtatc aaggagagaa atcattggat catatggtaa gagtatgtac 1080
   acttttatag gaaactgcta agctgcattc ctaagtggct gtaccattgt gccttcccat 1140
   cagcaatgaa tgagacttcc tattgttcca catcctcatc agaatttggt gttgtcactg 1200
   atctgaattt tttccattgt aacagatgtg tagtggtatc tcactgttgt tttaatttgc 1260
   aatttcctaa tgacatatga tgttgaacat ctttttatat gcttacttgc catcagtgta 1320
   tcctctgatg aggtgtttgt gtagggcttt ggcccatttt taaatcaggt tatttatcct 1380
   cttattatta acttttaaga gtttagttct ttgcatattt tggataacaa tcctttatca 1440
   catatttctt ttgcaaattt ttctccagta tatggcttgt cttcttctcc tggcattgtc 1500
   cttctcagag cagaagtttt taattttaat aaactccagc ttataaatta tttatttcat 1560
   ggattgtgcc tttggttttg tacttaaaaa gtcattgtca tacctaaggt catctaggtt 1620
   ttctcctacg ttatctccta ggtgttttat agttttgcat tttacattta tatgtatgat 1680
   cagttttgag ttaattttta tgaagtgtgt aaggtttgta tctacattca ttttttgcat 1740
   gtggatgtcc atttgttcca gcaatatttg ttgaaaagac tatacttgct ctattgtatt 1800
   gtgtttcttt tttgtcaaag atcaattgac taaatttatg tgcgtcagtt tctgatctct 1860
   ctggtccatt gatatatttg tctattcttt caccaatacc acatagtcta gactactgta 1920
   gttgtatatg tcttgaagtc aggtagtgtt gatcctccaa ttttgttctc caatattgag 1980
   ttggctattg tgggtctttt gcttgcccat agaatcaatt agtcaatatt tacaaaataa 2040
   cttgctcgaa ctttgactgg gattaatcta taaatcaagt tgggaataag tgacattttg 2100
   acattatgga gtctttctga ccatgaacac aaactattga tccatttatt tagttatttg 2160
   atatctttca ccagagtttt tttgtttttt tcttatagat cttatacata ttttcttata 2220
   ttcatacctc agtattccat ttcagggtgt taatgtaaat ggtaatgtgt ttttaatttc 2280
   aaattccctt agttctttgc tggtatatag gaaagtgatt ggcttttgta tgttaacgtt 2340
   gtatcctgct cacttgctat aactgcttat tagttccagg agctttttta ttgtttcttt 2400
   tggattttct aagagacaat tacattatca gtgaacaaac acgatttatt tcttccctcc 2460
   caatcagtat tcattttatt tatttattgt gtgttattgc attagctagg acttctaata 2520
   caatgttgaa aagcattggt gaaaggaaac atccttgctt tgttcctgat tttagctata 2580
   ggtttttgta gctgttcttt attaagttga ggatatcctt ctctattctt agtttgctga 2640
   gaatttttat catgaatagg tgtaggattt tgcctaatgt tttcttctgt atctattgat 2700
   atgatcatgt aatttttctt tatctattga tatgatctgt tgatgtgatg aactacatta 2760
   attgagtttt caaatgttga accagtcttg catatctgga ataaatcgga gttggtcatc 2820
   atgtataact ttgttacact ttgttgcatt tgattttgta atattttctt gagaattttt 2880
   acatctatgt tcataaaaga tatcggtcta cagttttctt tcctttcttg taatatctct 2940
   gtctggtttt gctattaagg taattctggc ttcacaaaat taattatgga gtcttccctc 3000
   tacttctagt ttctggaaga gattgtagag aatggatgta atttctttct taaatgtttg 3060
   acgaaaatca gcactgatct catctgggct tggtgctttc tgttttggaa ggttattaat 3120
   tatttattca atttctatag tggatatagg cctatattga ttggcaattt cttcttgtat 3180
   gacttttggt acattctatt tcaaggaatt ggttcatttc atgtaggttg ttaaattcgt 3240
   gggtatagct gttcataata ttcatttatt atcctttcaa tgttcatgag atcagtagtg 3300
   atgttccttc tttcatttct gatattcata atttgtgtat tctctctttg tttcttagcc 3360
   tggtgagagg cttataaatt ttattgattt tttgaagaat cactttttgg ttttgctgat 3420
   tttcttgctg ggattatagg cgtgagccac cacactcttg ctcatttttt ctatttttgt 3480
   tttccacact ttttctgcct ctgtggattt tacacagcat tttgtataat tcgatttcct 3540
   ctttttagca tagcaattat tttagtcttt aactttttaa aatcagttgc cctagatttt 3600
   tttctcccaa cattttggga ggccaagggg agaggatcac ttcaagccag gagtttgaga 3660
   ccagcctgag caacatagca aggcactatc tatacaaaca taattttaaa aagtccaggc 3720
   attagctagg actgtgcctg tagtcccagc tactcaggag gctgagatga gaagatcacc 3780
   ggagcctaga aatttgaggc tgcagtgggc tgtgatcatg tcacttcact cctgcctaga 3840
   agacagttag accctgcctc taaaataaac aagcaaataa ataaaaagaa aggaagaaaa 3900
   gaagagcaag ggcagcaaat agaaaatagt aataaatatg gtagctatta atccaactat 3960
   gtcaataatt accttaaatg ttagtggtct aaatatactg caatggactg aatgtttatg 4020
   tctcctcaaa atgtatatga tgaaatgtaa gctcccaaaa tgatgttatc aggggagagt 4080
   gtttttggga ggggattatg tcatgagggt ggaggcctta caaatggggt tagtgctata 4140
   aaagagacca cagagagctg ccttggtcct tctgccatgt gagggcactg tgaaattatg 4200
   gccatctatg aagaagtggg cccttattag acatcaaatc tgcaaatacc ttgatcttga 4260
   atttcccagc ctccagaact atgggaaata aatttctgtt gtttacaagt aaatcatttt 4320
   atgttatttt gttacagaag cccaaaaaga tgaagacata caccagatca ctccattctc 4380
   ttcttgcttg catggtttct gaggatacgt tggatgtaat tctaatattc tctataggta 4440
   ttttctttat ggctcctcta caggtaaggt gttttttccc tttggcttca tttaagaatt 4500
   tttcgttaac ctttgatttt ctgaagtgtg aatatgttat gcctaggtat catttgtttg 4560
   tttgggtttt cttggcatat atcttcctga tgttctctga acttccagaa tctgtgattt 4620
   gttgtctgac attaatttgg aggaattttt ggtattattg ctttaaatat tgcttctgct 4680
   cctttttctc tttctttgac ttacagtatt cccattacat gtaattatct cacagttctt 4740
   gaaagttttg ttttgttctt tttgttagtc gtttttcttt ctgtttttca gttttggcag 4800
   tttctgttta cgtatcttca atctcagaaa ttctttcctc aagcatgttc agcccactaa 4860
   tgtgtacttc aaaagtattc tacatttctt ttacagtgtt tttgatctct agaattttta 4920
   aattctttct taaaactttc atctctcagg aattcaagac tagcctgggc aacatagtga 4980
   aactctatct ctacaaaaca ttagccaggt atggtgatgc atgcctgtag tcagagctac 5040
   tcaggaggct aaggtgggag gatcacctga gcctgggaag ttgaggttgc agtgagccaa 5100
   ggtcacgcca ctgcactctg gattgggcaa cagagccaga ccctgtctca aaaaaaagaa 5160
   aaattccatg gctctgctta cattatccat ctgatcttac atgttgccta ttttttccat 5220
   taaaactcct agcctattaa tcatagtttt tttataatta atactccgat gtgataatgt 5280
   cttagtccaa ttactgtggt tataacagaa tgccacaaac tgggtgattt ataaacaaaa 5340
   gaagctgatt taggctgatt tagaggctgg ggagtccaag agcttggtgc tagcatctga 5400
   tgagtgtctt cttgcttcat cataacatgg gagagggcat cacgtgtgaa gagagcttac 5460
   tcttataaca tagccactcc cacaagaatt aacccaccgc catgagagcc atgtgaattc 5520
   attcatgagg acagcgggtt aagtttccaa tatatggact tttcggggac acattcaaac 5580
   cacagcagtt agttgtaacg ttcgtgtcat gtctcattct ggttctgatg cttgtgcagt 5640
   ctcttcaaac tgcgtctttg ccttttagtg tgccttgcaa tgtggaaatg atatactggg 5700
   taagaggagc tgtagtaaag aggcttctag tgacgtagtg acaagctgtg ggagagggga 5760
   gtgttgcaca gtcctgccgc atgtcacagt cttccagtga gcctgtgtcc ctggactgtg 5820
   aacttcatgc ttgcttctca gcttccccag ccccttagat ggtacagaac tgttggaggg 5880
   gggtggagtt gtatatttcc cttgctctgg gtaggtcacc ctctgataaa acaccaggtt 5940
   aggcctctgg tgaaataatt tctcctgagg gcagaccttc tattaataat agaatgttcc 6000
   aacctatttc aaaatggttc ctcttctcct tccactgcca gaagcataat gagattttcc 6060
   ccctaatatt cgtggtaagg acctagcaga gctccaggag gtaacactct caagtgtctc 6120
   atactaccct gcaccatgac tgggctctgc tggagttctt aatttgcaga actgcccaca 6180
   ctgagcctcc cgcaatttct caattacagg gcaaactttc ccagccggca ctgggtcctt 6240
   ggaggtttct gtctgctggt ttcttcctct ggaggttgtg cttctgtgtt tgcctgtctc 6300
   tccaatttgg ggggcagtgg tttgcccaat gacctcaatt ctctgaaaga gctaagaaga 6360
   ggtgttaatt tttcggtttg ctcagctttc tacttgttgc tagaatggag cgccaatagt 6420
   gcctcctata gtgacatgta accctcaact ctagagatga tgaagcatac taatgacaaa 6480
   ggagaaatgc ttcagcagtt ttctgtcagc acattacccc ttgaaaaagc tgcttcttcc 6540
   acattctgca agagatgggt ctcaactcag agctcaaggc aaatgacttc cttcaaggag 6600
   aaggaataaa cagtctcaga aaccatgaaa gcctgccccc aggagtgtcc ctgaacctca 6660
   gcaggggcca cacttacctt gcagaaatag gtgaggcatg ctcctggtac aaaatcccaa 6720
   tggtacagaa ggacaaattg aaaaacaagt ctccctctaa acccctgacc ccgagctacc 6780
   tagttctcct ccctagaggc aaagctgtta ccagattctt gtatctcctt aacatatatc 6840
   cttagaagag ctgtcaagtg aacacatgtt taagtgaaaa cctattttag aagtgcattt 6900
   tcttaaggaa ctttagggtt ggaaggaacc tgtgtcagtc cttaattcac aacctccatt 6960
   agtacttatt gttcttgcac aaaaatcttt ctcaaaaaag ccctttccac tctgacatag 7020
   cttattctac ttttacttag ctccaataac ttataaaaca tatttttgaa agtctaaaat 7080
   ctgccactat gttttttttt cctaatcaat ctttactttg acctctaagc cagagaaaac 7140
   aggtggtcaa atgccttttg cctaagatgg aacttagaat atttgaagac ctcagatctt 7200
   caccctgcca aataacgtgt ttctcctccc ctttcacaga gcatttggtt ttaggaaatt 7260
   cagagccaca ttccttatag acaagactaa actcttattc aacatactca gaaacttctt 7320
   ctaagaggat aaccactcat cagaggaaaa aagtttctca tgtacagctg gcaaagggat 7380
   ggaaccatct gtgttattaa aattgacaga cgcttatgag atttattaag ggaaatacta 7440
   gagtcttagt acatacttgc taatatagca tacatgaagg ctttatctat aatttttttg 7500
   gccaagcaga aattttggta ttactcaccc taacaaattt ccaagacatt atgaaataga 7560
   attttaggtc ctgacatcac catttgtctc aggttttgaa gcgttgctgg acaagagggg 7620
   taaaacacgg ctctgccttg gattcaaagt tggcctctca tactagcaag tataccttgg 7680
   tatcctggtc acttctcccg gccacagcat cacattgcta taaaaggcag atacaagtat 7740
   taaccagctc acaggttatc agataagctt agtctgacca atgcttaaca cagcaactgg 7800
   gccactattg tcattcctgt ggtggtggca cacacaccca gcctctgtcc gggccatggt 7860
   ctaggaccac cctccacaga ggctgtgagc tagagcccta actgtgcagg gccctaacta 7920
   tgccaggcta cttatctctc ttaagaggac ttcattagtg cctgctcggc catacagttt 7980
   tttacttacc aagtaacaca gttatcagca cactccaggt actagccaag gactacaaaa 8040
   tcaacgtgaa tgtcagcttt tgtatcaaaa gctcaaagga gaaactcaaa ctttacatag 8100
   atgtcccatg aagatgttca gcaaacccat tcttctctgt tccctggaat ccatcccagt 8160
   attgtgctat gtgtgtgtct agtaattctt tacaaaaagc tctgtttctt gtgatgctat 8220
   cagatcacat tgaagaatat acaagccgta ctatgaaggc tgttgtctca tatagtccta 8280
   acgtagtgag aactgatgtt cttacatgct gtctttttgg gcactcaaag aaattcctgt 8340
   acagtcttac aaatcagttg tagcttaaat tgatttgtgt tgtgacttgt acacacaggt 8400
   cacattccct tgacagaaaa tatagtttaa aaccaaattt gcagcccttg ttaagtgaat 8460
   gcacaggact ttattgtatt caggtctttt attgtaagac tcactcctgt cttcatttta 8520
   tgttccactg ttgtgcttcc catttgcctt tctctagttt tgttttctgt gtttctacgg 8580
   actgctctca gcccaggtgt gcaggaagca cacacatgcc tgcagagcct tcatggcctc 8640
   tgcattcagg gcatgacttc aacgcacagt ggctgtactg atttgttaaa acaaaggaac 8700
   agattacttc tcctaattca cagggaagtt ccaggttgtg cgggcagtga gcagacctgt 8760
   gtctgtctgc gcttgccctg gtgaaaaacc ccaccgttca ggctgcaggg tgcgagaccc 8820
   aggcacaaac attttgctgg atgaggagga aagatgtaag gttgctcccc ttcagagaca 8880
   gcaaagggca ggtctgtagc ttcacttact tcaggattgt gatttttgac agagccgaga 8940
   gatcagggtt gttgaaccag gcctgaaggt cctagtgaat ctcgtgaaga gaggaggggt 9000
   ctggctgtaa catggaccta gaggacattt ttactgcagg agaaggaaca gtggggatgg 9060
   ggtggacttg ccaaaggaat atagctcaag ttcctgcagc ccaaaaaagc tcagtttctt 9120
   ttggccaaag cttccgcgag tttccctggc atttctcctg cgggagctac aggggcagtg 9180
   ggacacttag cctctctaaa agcacctcca cggctgtttg tgtcaagcct ttattccaag 9240
   agcttcactt ttgcgaagta atgtgcttca cacattggct tcaaagtacc catggctggt 9300
   tgcaataaac attaaggagg cctgtctctg cacccggagt tgggtgccct catttcagat 9360
   gatttcgagg gtgcttgaca agatctgaag gaccctcgga ctttagagca ccacctcgga 9420
   cgcctggcac ccctgccgcg cgggcacggc gacctcctca gctgccaggc cagcctctga 9480
   tccccgagag ggtcccgtag tgctgcaggg gaggtgggga cccgaataaa ggagcagttt 9540
   ccccgtcggt gccattatcc gacgctggct ctaaggctcg gccagtctgt ctaaagctgg 9600
   tacaagtttg ctttgtaaaa caaaagaagg gaaaggggga aggggaccct ggcacagatt 9660
   tggctcgacc tggacatagg ctgggcctgc aagtccgcgg ggaccgggtc cagaggggca 9720
   gtgctgggaa cgcccctctc ggaaattaac tcctcagggc acccgctccc ctcccatgcg 9780
   ccgccccact cccgccggag actaggtccc gcgggggcca ccgctgtcca ccgcctccgg 9840
   cggccgctgg ccttgggtcc ccgctgctgg ttctcctccc tcctcctcgc attctcctcc 9900
   tcctctgctc ctcccgatcc ctcctccgcc gcctggtccc tcctcctccc gccctgcctc 9960
   cccgcgcctc ggcccgcgcg agctagacgt ccgggcagcc cccggcgcag cgcggccgca 10020
   gcagcctccg ccccccgcac ggtgtgagcg cccgacgcgg ccgaggcggc cggagtcccg 10080
   agctagcccc ggcggccgcc gccgcccaga ccggacgaca ggccacctcg tcggcgtccg 10140
   cccgagtccc cgcctcgccg ccaacgccac aaccaccgcg cacggccccc tgactccgtc 10200
   cagtattgat cgggagagcc ggagcgagct cttcggggag cagcgatgcg accctccggg 10260
   acggccgggg cagcgctcct ggcgctgctg gctgcgctct gcccggcgag tcgggctctg 10320
   gaggaaaaga aaggtaaggg cgtgtctcgc cggctcccgc gccgcccccg gatcgcgccc 10380
   cggaccccgc agcccgccca accgcgcacc ggcgcaccgg ctcggcgccc gcgcccccgc 10440
   ccgtcctttc ctgtttcctt gagatcagct gcgccgccga ccgggaccgc gggaggaacg 10500
   ggacgtttcg ttcttcggcc gggagagtct ggggcgggcg gaggaggaga cgcgtgggac 10560
   accgggctgc aggccaggcg gggaacggcc gccgggacct ccggcgcccc gaaccgctcc 10620
   caactttctt ccctcacttt ccccgcccag ctgcgcagga tcggcgtcag tgggcgaaag 10680
   ccgggtgctg gtgggcgcct ggggccgggg tcccgcacgt gcgccccgcg ctgtcttccc 10740
   agggcgcgac ggggtcctgg cgcgcacccg aggggcgggc gctgcccacc cgccgagact 10800
   gcactgttta gggaagctga ggaaggaacc caaaaataca gcctcccctc ggaccccgcg 10860
   ggacaggcgg ctttctgaga ggacctcccc gcctccgccc tccgcgcagg tctcaaactg 10920
   aagccggcgc ccgccagcct ggccccggcc cctctccagg tccccgcgat cctcgttccc 10980
   cagtgtggag tcgcagcctc gacctgggag ctgggagaac tcgtctacca ccacctgcgg 11040
   ctcccgggga ggggtggtgc tggcggcggt tagtttcctc gttggcaaaa ggcaggtggg 11100
   gtccgacccg ccccttgggc gcagaccccg gccgctcgcc tcgcccggtg cgccctcgtc 11160
   ttgcctatcc aagagtgccc cccacctccc ggggacccca gctccctcct gggcgcccgc 11220
   gccgaaagcc ccaggctctc cttcgatggc cgcctcgcgg agacgtccgg gtctgctcca 11280
   cctgcagccc ttcggtcgcg cctgggcttc gcggtggagc gggacgcggc tgtccggcca 11340
   ctgcaggggg ggatcgcggg actcttgagc ggaagccccg gaagcagagc tcatcctggc 11400
   caacaccatg gtgtttcaaa atggggctca cagcaaactt ctcctcaaaa cccggagact 11460
   ttctttcttg gatgtctctt tttgctgttt gaagaatttg agccaaccaa aatattaaac 11520
   ctgtcttaca cacacacaca cacacacaca cacacacaca ccggattgct gtccctggtt 11580
   caagtgtgcc aagtgtgcag acagaacatg agcgagtctg gcttcgtgac taccgaccat 11640
   aaacccactt gacaggggaa acatgccttg gaaggtttaa ttgcacaatt ccaaccttga 11700
   gctgcgcggg ttccaagagc caggcccgta cttgctgttg atgtcattgg cttggggagt 11760
   tggggtttgg tgcccagcgc ggtcgttggg ggaggggcaa ggcatagaac agtggttccc 11820
   agaccttgct gcacattgga attacctggg attaaaaaaa aaaaaatcaa aacaaaaacc 11880
   agtgtctggc tcccgccccc agacattctg atttaattgg catggggcaa gacctggact 11940
   tgggattttt tttaatgctc ttcatgtgat ctgttgggca gccagatttg gggatcacta 12000
   gacggaagaa ggattgttaa agtctccgga gatgttactt gccaatgcta agagctcttt 12060
   gaggacatct ggaattgtta caatattgcc aaatatagga aagagggaaa aggtagagtg 12120
   tgattccaat aataaaggat tccgcttttc attgaaggaa ctggtggaaa ggtttcttct 12180
   ctgctgagcc tgcaggcccg tcctgcctgc ctggggtgcc cgggagacgc gggcctgctc 12240
   cggagactgc tgactgccgg tcctgttagt caggtgtcag ccctgtctct gccgaagaga 12300
   ctcttctctt tattttaaat taaaccctca gagcaccacc aaagcatcac ttttctccct 12360
   ccattggtgt tctcattctt tgatgttact tgtttgaaca ccactattag tagttggaga 12420
   tttgttcctg agaaaaatat aaataccact taatttgcct gtttgtcccg cattcactca 12480
   aaacagaatg ctcctgaaga caagagagag agtaggagaa cagacgctat tccattacag 12540
   taacataaaa gactggattt tcaggggcaa attattaaaa taggagatga gctcttttaa 12600
   cagaaatttg tttaaggcct gtgtctatca aattcagtgg attttattca agatgcactt 12660
   tgtttagtgg gagttttgtt tggttctggg acatgctaac ttctagactt gctgctctta 12720
   gaggtaatga ctgccagaca ccatttcatg agtcctaatc cccacattaa gcataagagg 12780
   tgcacactct cctcctatgg gggaaactga ggtacgaaga actaaagtga ctttcccaca 12840
   gctggtggga ggcagacggg aaattcacac caggggcttc caactccaga tccctctctc 12900
   aacttccaaa ctccactgcc ttgtccgagt tctggtttca ggagatccaa atcaggtgtg 12960
   tgcaaatgtc taatgtcaga gctggcaagg ggaaagggcc 13000